(19) Europäisches Patentamt
European Patent Office
Office européen des brevets

(11) **EP 4 659 662 A1**

(12) **EUROPEAN PATENT APPLICATION**
published in accordance with Art. 153(4) EPC

(43) Date of publication:
**10.12.2025 Bulletin 2025/50**

(21) Application number: **24787815.0**

(22) Date of filing: **23.02.2024**

(51) International Patent Classification (IPC):
***A61B 5/0205*** (2006.01)

(52) Cooperative Patent Classification (CPC):
**A61B 5/00; A61B 5/0205; A61B 5/021;
A61B 5/024; A61B 5/0245; A61B 5/1455;
A61B 5/318**

(86) International application number:
**PCT/CN2024/078320**

(87) International publication number:
**WO 2024/212709 (17.10.2024 Gazette 2024/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.04.2023 CN 202310456048**

(71) Applicant: **Huawei Technologies Co., Ltd.
Shenzhen, Guangdong 518129 (CN)**

(72) Inventors:
• **ZHAO, Menglong
Shenzhen, Guangdong 518129 (CN)**

• **LIU, Zhuang
Shenzhen, Guangdong 518129 (CN)**
• **ZOU, Lin
Shenzhen, Guangdong 518129 (CN)**
• **WANG, Xu
Shenzhen, Guangdong 518129 (CN)**
• **LI, Weinan
Shenzhen, Guangdong 518129 (CN)**

(74) Representative: **Maiwald GmbH
Engineering
Elisenhof
Elisenstrasse 3
80335 München (DE)**

(54) **EXAMINATION APPARATUS AND ELECTRONIC DEVICE**

(57)   A detection apparatus and an electronic device are provided and relate to the field of terminal technologies. The detection apparatus includes: a support component (110), an optical receiver (120), an optical transmitter (130), and an FPC (140). A first groove (1101) and a second groove (1102) are provided on a bottom end surface of the support component (110), the bottom end surface of the support component (110) is connected to the FPC (140), to form a first accommodating cavity (150) corresponding to the first groove (1101) and a second accommodating cavity (160) corresponding to the second groove (1102), a first light exit window (1103) and a first light entry window (1104) are provided on a top end surface of the support component (110), the first groove (1101) communicates with the first light exit window (1103), and the second groove (1102) communicates with the first light entry window (1104). The optical transmitter (130) and the optical receiver (120) are both electrically connected to the FPC (140), the optical transmitter (130) is disposed in the first accommodating cavity (150), and the optical receiver (120) is disposed in the second accommodating cavity (160). A first electrode (141) is disposed on the FPC (140), and the first electrode (141) is electrically connected to the support component (110). The detection apparatus disposed as above resolves a problem that a structure design of an ECG electrode and a PPG module occupies an excessively large proportion of space of an entire device.

FIG. 3

## Description

This application claims priority to Chinese Patent Application No. 2023104560482, filed with the China National Intellectual Property Administration on April 14, 2023 and entitled "DETECTION APPARATUS AND ELECTRONIC DEVICE", which is incorporated herein by reference in its entirety.

## TECHNICAL FIELD

This application pertains to the field of terminal device technologies, and in particular, to a detection apparatus and an electronic device.

## BACKGROUND

With development of terminal devices such as a smartwatch, a smart band, smart glasses, and a headset, more terminal devices are with a health detection function. Specifically, a photoplethysmograph (photoplethysmograph, PPG) module and an electrocardiograph (electrocardiograph, ECG) electrode may be disposed in a terminal device to detect blood pressure of a wearer. A principle of measuring the blood pressure through the PPG module and the ECG electrode is as follows: First time of contraction of a specific blood vessel of the wearer may be measured through the PPG module, second time of contraction of a ventricle of the wearer may be measured through the ECG electrode, and pulse transit time (Pulse Transit Time, PTT) may be obtained based on a time interval between the first time and the second time. Shorter PTT time indicates a faster pulse wave velocity (Pulse Wave Velocity, PWV) and higher blood pressure. On the contrary, longer PTT time indicates lower blood pressure.

In an actual structure design of the terminal device, the ECG electrode and the PPG module are usually separately disposed. As a result, a structure design of the ECG electrode and the PPG module occupies an excessively large proportion of space of the entire terminal device. This is not conducive to miniaturization development of the terminal device.

## SUMMARY

This application provides a detection apparatus and an electronic device, to resolve a problem that a structure design of an ECG electrode and a PPG module occupies an excessively large proportion of space of an entire device to some extent. This implements an integrated design of the ECG electrode and the PPG module, saves stacking space of the entire device, and ensures a miniaturized and light-and-thin design of a terminal device.

To achieve the foregoing objective, this application uses the following technical solutions.

According to a first aspect, this application provides a detection apparatus. The detection apparatus includes a support component, an optical receiver, an optical transmitter, and an FPC.

A first groove and a second groove are provided on a bottom end surface of the support component, the bottom end surface of the support component is connected to the FPC, to form a first accommodating cavity corresponding to the first groove and a second accommodating cavity corresponding to the second groove, a first light exit window and a first light entry window are provided on a top end surface of the support component, the first groove communicates with the first light exit window, and the second groove communicates with the first light entry window.

The optical transmitter and the optical receiver are both electrically connected to the FPC, the optical transmitter is disposed in the first accommodating cavity, and the optical receiver is disposed in the second accommodating cavity.

A first electrode is disposed on the FPC, and the first electrode is electrically connected to the support component.

In the detection apparatus provided in this application, the first accommodating cavity configured to accommodate the optical transmitter and the second accommodating cavity configured to accommodate the optical receiver are separately provided on one side of the support component, and the light exit window and the light entry window that respectively correspond to the first accommodating cavity and the second accommodating cavity are provided on another opposite side of the support component, so that the optical transmitter and the optical receiver are disposed in the detection apparatus, and a function of collecting an electrical signal through the first electrode in the detection apparatus is implemented through the electrical connection between the support component and the first electrode on the FPC. The optical receiver, the optical transmitter, and the first electrode are integrated into the detection apparatus by fully using the support component. This avoids a structure design in which an ECG electrode and a PPG module (namely, the optical receiver and the optical transmitter) are separated, and implements a miniaturized and integrated structure design of the optical receiver, the optical transmitter, and the first electrode. The foregoing design effectively reduces space occupied by the optical receiver, the optical transmitter, and the first electrode in a terminal device, and resolves a problem that the structure design of the ECG electrode and the PPG module occupies an excessively large proportion of space of the entire device.

In addition, if the detection apparatus provided in this application is used in a wearable device such as a smartwatch or a smart band, after wearing the wearable device on which the detection apparatus is disposed, a user only needs to place a finger on the top end surface of the support component in the detection apparatus, so that an electrical signal corresponding to the ECG electrode and a PPG signal corresponding to the PPG module can be measured by the detection apparatus at the same time, and then detection of a health parameter such as blood pressure can be implemented. In this structure design, the electrical signal and the PPG signal do not need to be separately obtained in sequence, thereby simplifying operation steps of health detection, and effectively improving product practicability.

In a possible implementation, the support component may be integrally formed into an integrated structure, or the support component may be formed by splicing a plurality of mechanical parts. For example, the support component includes a base and a first mechanical part, the first groove and the second groove are provided on a bottom end surface of the base, a top end surface of the base is fastened to a bottom end surface of the first mechanical part, and the first light exit window and the first light entry window are provided on the first mechanical part. The first light exit window communicates with the first groove, and the first light exit window is used for emission of a light ray emitted by the optical transmitter disposed in the first groove. The first light entry window communicates with the second groove, and the first light entry window is configured to receive a reflected light ray by the optical receiver disposed in the second groove.

In a possible implementation, transmittance of the first light exit window and/or transmittance of the first light entry window are/is greater than 90%. In other words, both the first light exit window and the first light entry window may be made of materials whose transmittance is greater than 90%. In this way, the optical signal transmitted by the optical transmitter can be effectively irradiated to a human body tissue and blood, and the optical signal reflected by the human body tissue and the blood is received by the optical receiver, to further accurately obtain blood flow data and data of contraction of a blood vessel corresponding to the PPG module, thereby ensuring accuracy of health measurement.

In a possible implementation, a conductive portion is disposed on the support component, and the support component is electrically connected to the first electrode through the conductive portion.

Based on the foregoing optional implementation, the electrical connection between the support component and the first electrode is directly implemented by the conductive portion disposed on the support component. This helps simplify a structure design of the electrical connection between the support component and the first electrode. In addition, the conductive portion may be disposed at a plurality of locations on the support component, and manufacturing is simple and convenient. For example, the conductive portion may be disposed between the light entry window and the light exit window of the support component, or the conductive portion is disposed at one end that is of the support component and that is away from the light exit window, or the conductive portion is disposed at one end that is of the support component and that is close to the light exit window.

In a possible implementation, the detection apparatus further includes an elastic component, a third accommodating cavity configured to accommodate the elastic component is provided on the support component, and the support component is electrically connected to the first electrode through the elastic component disposed in the third accommodating cavity.

Based on the foregoing optional implementation, disposition locations and disposition manners of the elastic component and the third accommodating cavity in the detection apparatus are flexible. For example, the disposition locations of the elastic component and the third accommodating cavity in the detection apparatus may be between the light entry window and the light exit window of the support component, or may be on two sides of the light entry window and the light exit window.

For example, a manner in which the elastic component is separately electrically connected to the support component and the first electrode may be: directly bonding two ends of the elastic component to the support component and the first electrode respectively through a conductive adhesive; or may be: soldering two ends of the elastic component between the support component and the first electrode; or may be: connecting one end of the elastic component to the elastic component and the support component through a conductive adhesive, and connecting the other end of the elastic component to the elastic component and the first electrode on the FPC through tin soldering; or may be: connecting one end of the elastic component to the first electrode on the FPC through tin soldering, and implementing the electrical connection through direct rigid contact between the other end of the elastic component and the support component.

In a possible implementation, a first through hole corresponding to the first electrode is provided on the support component, a first conductive adhesive is disposed in the first through hole, and the support component is electrically connected to the first electrode through the first conductive adhesive.

Based on the optional manner, a design solution in which the support component is electrically connected to the first electrode is further enriched. According to different materials of the support component, the electrical connection between the support component and the first electrode may be flexibly implemented by using the method. For example, if the support component is made of a metal light-shielding material, a through hole may be provided on the support component, and a first conductive adhesive is injected into the through hole. After being solidified, the first conductive adhesive may be used for the electrical connection between the support component and the first electrode. For another example, if the support component is made of a plastic light-shielding material, a through hole may be provided on the support component, a first conductive adhesive is injected into the through hole, and a surface of the support component is covered with a transparent conductive coating. The transparent conductive coating is connected to the first conductive adhesive. In this way, the electrical connection between the support component and the first electrode may also be implemented.

In addition to the foregoing several possible implementations, the surface of the support component may alternatively be directly covered with a conductive coating or a conductive adhesive. When the FPC is connected to the bottom end surface of the support component, the support component and the first electrode are electrically connected by covering the conductive coating or the conductive adhesive on the surface of the support component. In this way, a material of the support component during actual application does not need to be considered; and in an actual manufacturing process, a specific disposition manner of the conductive coating or the conductive adhesive on the support component and the first electrode that are electrically connected may be flexibly changed.

In a possible implementation, the support component includes a support component body, and the support component body is made of a conductive light-shielding material.

It should be understood that, based on the possible implementation, the support component and the support component body are of a same structure. In other words, the support component is made of a conductive light-shielding material, and the support component body is also made of a conductive light-shielding material.

In an actual design, to reduce abrasion of the support component body and better protect the support component body, a surface of the support component body may also be coated with a conductive material. It should be understood that when the support component body is made of a conductive light-shielding material, to reduce a thickness of the support component body, a first conductive coating may not be coated on the surface of the support component body.

In a possible implementation, the support component includes a support component body and a first conductive coating disposed on a surface of the support component body, and the support component body is made of a light-shielding insulation material.

For example, the first conductive coating may be a transparent conductive adhesive, a non-transparent conductive adhesive, a conductive paint, or the like.

When the support component body is made of a light-shielding insulation material, to implement the electrical connection between the support component and the first electrode, the surface of the support component body may be coated with the first conductive coating. Certainly, in a feasible implementation, regardless of whether the support component body is made of a conductive light-shielding material or a light-shielding insulation material, the surface of the support component body may be coated with the first conductive coating.

Optionally, the conductive light-shielding material may be a material such as iron, copper, aluminum, titanium alloy, or liquid metal.

Optionally, the light-shielding insulation material may be black plastic, rubber, blackened glass, light-shielding ceramic, or the like.

In a possible implementation, the first conductive coating may be light-transmitting.

During actual application, the first conductive coating may be light-transmitting, or may be light-shielding. This facilitates selection of a material of the first conductive coating, reduces design difficulty, and simplifies structure disposition.

In a possible implementation, an orthographic projection of the first conductive coating on the support component body covers the first light exit window and/or the first light entry window. In other words, when the first conductive coating is light-transmitting, the first conductive coating not only may be coated on the support component body, but also may be disposed on the first light exit window and/or the first light entry window on the top end surface of the support component. In this disposition manner, a contact area between skin and the first conductive coating on the support component can be effectively increased, thereby improving efficiency of collecting an electrical signal by the first electrode.

On the contrary, when the first conductive coating is light-shielding, the orthographic projection of the first conductive coating on the support component body does not cover the first light exit window and/or the first light entry window. In this way, transmission of a light ray in the first light exit window and/or the first light entry window is not affected by disposition of the light-shielding first conductive coating.

In a possible implementation, the light exit window is a second through hole provided on the support component, and/or the light entry window is a third through hole provided on the support component.

In a possible implementation, a first transparent part is disposed in the second through hole, and/or a second transparent part is disposed in the third through hole.

Optionally, the first transparent part and/or the second transparent part may be a transparent adhesive, transparent plastic, or the like.

Based on the foregoing optional implementation, the optical transmitter disposed in the first accommodating cavity and/or the optical receiver disposed in the second accommodating cavity may have a waterproof, mould-proof, dustproof, and protection functions. This further extends a function of the detection apparatus, and improves practicability of the detection apparatus.

In a possible implementation, the support component includes a support component body and a conductive light-shielding layer disposed on a surface of the support component body, and the support component body is made of a light-transmitting material.

Optionally, the conductive light-shielding layer includes any one of a silkscreen, conductive ink, a conductive adhesive, a black matrix, or a light-shielding sheet.

In a possible implementation, the support component includes a support component body, a light-shielding insulation layer disposed on a surface of the support component body, and a second conductive coating disposed on a surface of the light-shielding insulation layer, and the support component body is made of a light-transmitting material.

Optionally, the light-shielding insulation layer may be a silicone layer to which a dye is added, a coating sprayed with extinction ink or black ink, or another light-shielding component.

Based on the foregoing embodiment, the light-transmitting material may be a conductive light-transmitting material, or may be a light-transmitting insulation material. For example, the conductive light-transmitting material includes indium tin oxide or the like, and the light-transmitting insulation material includes transparent glass, transparent plastic, or the like.

In a possible implementation, the second conductive coating may be light-transmitting.

Optionally, the second conductive coating may alternatively be a transparent conductive adhesive, a non-transparent conductive adhesive, a conductive paint, or the like.

In a possible implementation, an orthographic projection of the second conductive coating on the support component body covers the first light exit window and/or the first light entry window.

Based on the foregoing optional implementation, when the second conductive coating is a conductive light-transmitting coating, the second conductive coating is disposed in the first light exit window and/or the first light entry window of the support component, so that a contact area between skin and the support component can be increased, thereby reducing a case in which an electrical signal cannot be obtained through the support component and the first electrode that are electrically connected due to a small contact area between the skin and the support component. On the contrary, when the second conductive coating is light-shielding, the orthographic projection of the second conductive coating on the support component body does not cover the first light exit window and/or the first light entry window. In this way, transmission of a light ray in the first light exit window and/or the first light entry window is not affected by disposition of the light-shielding second conductive coating.

According to a second aspect, this application further provides another detection apparatus. The detection apparatus includes a support component, an optical receiver, an optical transmitter, and an FPC.

The support component includes a first mechanical part and a base, the first mechanical part is fastened to the base, a first groove and a second groove are provided on a bottom end surface of the base, a second light exit window and a second light entry window are provided on the first mechanical part, the first groove communicates with the second light exit window, and the second groove communicates with the second light entry window.

The base is fastened to the FPC, and forms a first accommodating cavity and a second accommodating cavity with the FPC, the first accommodating cavity corresponds to the first groove, the second accommodating cavity corresponds to the second groove, the optical transmitter is disposed in the first accommodating cavity, and the optical receiver is disposed in the second accommodating cavity.

The optical transmitter and the optical receiver are both electrically connected to the FPC, the optical transmitter is disposed in the first accommodating cavity, and the optical receiver is disposed in the second accommodating cavity.

A first electrode is disposed on the FPC, and the first electrode is electrically connected to the first mechanical part through the base.

Based on the foregoing optional implementation, the support component may not only be made of an integrated structure, but also be formed by splicing a plurality of structure bodies. In this way, a structure design, processing, and assembly process of the support component is enriched, and such structure design can provide wide selection for a specific material of the support component in a design process of the detection apparatus.

It should be noted that, the optical receiver, the optical transmitter, and the FPC in the detection apparatus according to the first aspect and the optical receiver, the optical transmitter, and the FPC in the detection apparatus according to the second aspect are disposed to be of a same specific structure, and a difference is that the support component in the detection apparatus according to the first aspect and the support component in the detection apparatus according to the second aspect are of different specific structures. The support component in the detection apparatus according to the first aspect is of an integrated structure, and the support component in the detection apparatus according to the second aspect is formed by two mechanical parts: the first mechanical part and the base.

In a possible implementation, a conductive portion is disposed on the base, and the first mechanical part is electrically connected to the first electrode through the base and the conductive portion.

In a possible implementation, the detection apparatus further includes an elastic component, a third accommodating cavity configured to accommodate the elastic component is provided on the base, and the first mechanical part is electrically connected to the first electrode through the elastic component disposed in the third accommodating cavity.

In a possible implementation, a first through hole corresponding to the first electrode is provided on the base, a first conductive adhesive is disposed in the first through hole, and the first mechanical part is electrically connected to the first electrode through the first conductive adhesive.

In a possible implementation, the first mechanical part includes a first mechanical part body, and the first mechanical part body is made of a conductive light-shielding material.

In a possible implementation, the first mechanical part includes a first mechanical part body and a first conductive coating disposed on a surface of the first mechanical part body, and the first mechanical part body is made of a light-shielding insulation material.

In a possible implementation, the first conductive coating disposed on the surface of the first mechanical part body may be light-transmitting.

In a possible implementation, an orthographic projection of the first conductive coating on the first mechanical part body covers the second light exit window and/or the second light entry window.

In a possible implementation, the first mechanical part includes a first mechanical part body and a conductive light-shielding layer disposed on a surface of the first mechanical part body, and the first mechanical part body is made of a light-transmitting material.

In a possible implementation, the first mechanical part includes a first mechanical part body, a light-shielding insulation layer disposed on a surface of the first mechanical part body, and a second conductive coating disposed on a surface of the light-shielding insulation layer, and the first mechanical part body is made of a light-transmitting material.

In a possible implementation, the second conductive coating disposed on the surface of the light-shielding insulation layer may be light-transmitting.

In a possible implementation, an orthographic projection of the second conductive coating on the first mechanical part body covers the second light exit window and/or the second light entry window.

In a possible implementation, the base includes a base body, and the base body is made of a conductive light-shielding material.

In a possible implementation, the base includes a base body and the first conductive coating disposed on a surface of the base body, and the base body is made of a light-shielding insulation material.

In a possible implementation, the first conductive coating disposed on the surface of the base body may be light-transmitting.

In a possible implementation, an orthographic projection of the first conductive coating on the base body covers the second light exit window and/or the second light entry window.

In a possible implementation, the base includes a base body and a conductive light-shielding layer disposed on a surface of the base body, and the base body is made of a light-transmitting material.

In a possible implementation, the base includes a base body, the light-shielding insulation layer disposed on a surface of the base body, and the second conductive coating disposed on the surface of the light-shielding insulation layer, and the base body is made of a light-transmitting material.

In a possible implementation, the second conductive coating disposed on the surface of the light-shielding insulation layer may be light-transmitting.

In a possible implementation, an orthographic projection of the second conductive coating on the base body covers the second light exit window and/or the second light entry window.

In a possible implementation, the second light exit window is a second through hole provided on the first mechanical part, and/or the second light entry window is a third through hole provided on the first mechanical part.

In a possible implementation, a first transparent part is disposed in the second through hole, and/or a second transparent part is disposed in the third through hole.

In a possible implementation, transmittance of the second light exit window and/or transmittance of the second light entry window are/is greater than 90%.

In a possible implementation, the detection apparatus further includes a key Dome sheet and a support structure, the FPC is disposed around the support structure, the key Dome sheet is disposed on a first surface of the FPC, the optical receiver is disposed on a second surface of the FPC, and the first surface and the second surface are disposed opposite to each other.

Optionally, a shape of the support structure may be a cuboid, a cube, or another polyhedron.

Optionally, a material of the support structure may be a hard material such as a steel plate.

Based on the foregoing optional implementation, the optical receiver (or the optical transmitter) and the key Dome sheet are respectively disposed on the two opposite surfaces of the FPC. After the detection apparatus is disposed in a terminal device or another electronic device, it is convenient to use the detection apparatus to perform different function settings. For example, after the detection apparatus is disposed in a smartwatch, not only the detection apparatus may be used to detect blood pressure, blood oxygen saturation, a heart rate, and the like of a wearer, but also the wearer may press the detection apparatus disposed on the smartwatch, so that a display interface of the smartwatch goes to interfaces such as a health management interface or a voice broadcast health detection result interface. In this design manner, an application scope of the detection apparatus can be further enriched, and practicability of the detection apparatus is improved.

Optionally, a thickness of the support structure is 0.2 millimeter.

In a possible implementation, the detection apparatus is of an integrated structure. In this way, not only a processing process for separately manufacturing a device such as a support component or an FPC can be reduced, processing difficulty can be reduced, processing efficiency can be improved, and manufacturing process costs can be reduced, but also the detection apparatus can have stronger mechanical rigidity, and stability of the detection apparatus can be improved.

Optionally, the detection apparatus may be injection-molded into an integrated structure in a low temperature and low-pressure injection molding manner.

Optionally, a side length of a long side edge of the support component is 11.55 millimeters, a side length of a short side edge of the support component is 2.8 millimeters, and a thickness of the support component is 2.2 millimeters.

Optionally, a thickness of the FPC is 0.12 millimeter.

Optionally, a thickness of the light exit window and/or a thickness of the light entry window are/is 0.2 millimeter.

Optionally, a side length of a long side edge of the optical transmitter is 1.85 millimeters, and a thickness of the optical transmitter is 0.6 millimeter; and a side length of a long side edge of the optical receiver is 2.6 millimeters, and a thickness of the optical receiver is 0.6 millimeter.

According to a third aspect, this application provides an electronic device. The electronic device includes a housing and the detection apparatus according to any one of the first aspect and the possible implementations of the first aspect, and the detection apparatus is embedded in an outer surface of a side wall of the housing.

In a possible implementation, a second electrode is embedded on a bottom wall of the housing, and the second electrode and the first electrode are configured to form a path to collect an electrical signal when skin is separately in contact with the top end surface of the support component and the bottom wall of the housing.

According to the electronic device provided in this application, in comparison with the conventional technology in which a PPG module (an optical receiver and an optical transmitter) is disposed on the bottom wall of the housing of the electronic device, in this application, the detection apparatus is disposed on the side wall of the housing of the electronic device in an integrated design manner, so that a finger can be in contact with the support component in the detection apparatus, and a PPG signal collected by a finger part is more accurate than a PPG signal (namely, an optical signal) collected by another wearing part (for example, a wrist). In this way, the PPG module in the detection apparatus collects a more abundant and accurate PPG signal waveform, and accuracy of measuring a health parameter such as blood pressure is improved.

It should be understood that, in the foregoing possible implementation, the skin is electrically connected to the first electrode through contact between the skin and the top end surface of the support component, and the skin is electrically connected to the second electrode through contact between the skin and the bottom wall of the housing. In this way, the first electrode and the second electrode can communicate with each other through the skin, so that the first electrode and the connected second electrode that communicate with each other can collect the electrical signal of the skin.

In a possible implementation, the electronic device further includes a processor, and an FPC in the detection apparatus is electrically connected to the processor through a board-to-board BTB connector.

Optionally, a quantity of PINs of the BTB connector is 10.

It should be understood that the quantity of PINs of the BTB connector is related to a quantity of health detection functions disposed in the detection apparatus.

To facilitate actual use of the detection apparatus, optionally, the detection apparatus is disposed protruding from the housing.

According to a fourth aspect, this application provides another electronic device. The electronic device includes a housing, a support component, an optical receiver, an optical transmitter, and an FPC.

The FPC is disposed in the housing, and the optical receiver and the optical transmitter are both electrically connected to the FPC.

The support component is embedded on an outer surface of a side wall of the housing, and a light exit window corresponding to the optical transmitter and a light entry window corresponding to the optical receiver are provided on the support component.

A first electrode is further disposed on the FPC, and the first electrode is electrically connected to the support component.

A second electrode is embedded on a bottom wall of the housing, and the second electrode and the first electrode are configured to form a path to collect an electrical signal when skin is separately in contact with the top end surface of the support component and the bottom wall of the housing.

It should be understood that a specific structure of the support component may be a structure of the support component in the detection apparatus according to any one of the first aspect and the possible implementations of the first aspect. Certainly, the specific structure of the support component may also be a support plate embedded in the housing. A projection shape of the support plate on the FPC may be a rectangle, a square, a runway shape, an ellipse, any polygon, or the like. A light exit window and a light entry window are provided on the support plate, and there is at least one electrical connection path between a top end surface of the support plate and the first electrode.

In a possible implementation, a conductive portion is extended and disposed on the support component, and the top end surface of the support component is electrically connected to the first electrode through the conductive portion.

In a possible implementation, the electronic device further includes an elastic component. One end of the elastic component is electrically connected to a bottom end surface of the support component, and the other end of the elastic component is electrically connected to the first electrode. When skin is in contact with the top end surface of the support component, the top end surface of the support component is electrically connected to the first electrode through the support component and the elastic component, and the first electrode may collect an electrical signal of the skin through the foregoing electrical connection path.

Optionally, to avoid a case in which detection data is inaccurate because an optical signal transmitted by the optical transmitter and an optical signal received by the optical receiver affect each other, a light-blocking component may be further disposed between two sides of the elastic component. The light-blocking component is configured to separate the optical signal transmitted by the optical transmitter from the optical signal received by the optical receiver.

For technical effect of the second aspect to the fourth aspect provided in this application, refer to technical effect of the possible implementations of the first aspect. Details are not described herein again.

## BRIEF DESCRIPTION OF DRAWINGS

FIG. 1 is a diagram of a relationship between blood pressure BP and pulse transit time PTT according to an embodiment of this application;

FIG. 2 is a diagram of a structure of a side surface of a detection apparatus disposed in a smartwatch according to an embodiment of this application;

FIG. 3 is a diagram of a cross-sectional structure of a detection apparatus according to an embodiment of this application;

FIG. 4 is a diagram of a top-view structure of a detection apparatus according to an embodiment of this application;

FIG. 5 is a diagram of a cross-sectional structure of a first conductive coating disposed on a top end surface of a support component body according to an embodiment of this application;

FIG. 6 is a diagram of a cross-sectional structure of a first conductive coating disposed on a surface of a support component body according to an embodiment of this application;

FIG. 7 is a diagram of a cross-sectional structure of a light-shielding insulation layer and a second conductive coating disposed on a surface of a support component body when the second conductive coating is light-transmitting according to an embodiment of this application;

FIG. 8 is a diagram of a cross-sectional structure of a first electrode and a support component that are electrically connected through a conductive portion according to an embodiment of this application;

FIG. 9 is a diagram of a cross-sectional structure of a support component and a first electrode that are electrically connected through an elastic component according to an embodiment of this application;

FIG. 10 is a diagram of a cross-sectional structure of a first conductive coating disposed on a top end surface of a support component when a third accommodating cavity provided on the support component is a through hole according to an embodiment of this application;

FIG. 11 is a diagram of a cross-sectional structure of a first electrode and a support component that are electrically connected in a manner of injecting a first conductive adhesive into a first through hole according to an embodiment of this application;

FIG. 12 is a diagram of a cross-sectional structure of a support component formed by splicing two mechanical parts according to an embodiment of this application;

FIG. 13 is a diagram of a cross-sectional structure of a first mechanical part that is electrically connected to a first electrode through a base and a conductive portion according to an embodiment of this application;

FIG. 14 is a diagram of a cross-sectional structure of a first mechanical part that is electrically connected to a first electrode through an elastic component disposed in a fourth through hole according to an embodiment of this application;

FIG. 15 is a diagram of a cross-sectional structure of a first mechanical part and a first electrode that are electrically connected by injecting a first conductive adhesive into a first through hole according to an embodiment of this application;

FIG. 16 is a diagram of a structure of an integrated forming process of a detection apparatus according to an embodiment of this application;

FIG. 17 is a diagram of a structure of another integrated forming process of a detection apparatus according to an embodiment of this application;

FIG. 18 is a diagram of a cross-sectional structure of a detection apparatus part after a detection apparatus is disposed in an electronic device according to an embodiment of this application;

FIG. 19 is a diagram of a cross-sectional structure of a support component and a first electrode that are electrically connected through an elastic component after a detection apparatus is disposed in an electronic device according to an embodiment of this application;

FIG. 20 is a diagram of an actual application scenario in which a detection apparatus is disposed in a smartwatch according to an embodiment of this application; and

FIG. 21 is a diagram of a cross-sectional structure of a key Dome sheet and a support structure 190 that are disposed in a detection apparatus according to an embodiment of this application.

Reference numerals:

100: detection apparatus;
110: support component; 111: first mechanical part; 1111: second light exit window; 1112: second light entry window; 112: base; 1100: support component body; 1101: first groove; 1102: second groove; 1103: first light exit window; 1104: first light entry window; 1105: conductive portion; 1106: first conductive coating; 1107: light-shielding insulation layer; 1108: second conductive coating; 1109: first through hole; 1110: first conductive adhesive;
120: optical receiver;
130: optical transmitter;
140: FPC; 141: first electrode;
150: first accommodating cavity;
160: second accommodating cavity;
170: elastic component;
180: key Dome sheet;
190: support structure.

## DESCRIPTION OF EMBODIMENTS

To make the objectives, technical solutions, and advantages of embodiments of this application clearer, the following clearly and completely describes the technical solutions in embodiments of this application with reference to accompanying drawings in embodiments of this application. It is clear that the described embodiments are merely some rather than all of embodiments of this application. All other embodiments obtained by a person of ordinary skill in the art based on embodiments of this application without creative efforts shall fall within the protection scope of this application.

In the descriptions of embodiments of this application, the terms "first" and "second" are merely intended for a purpose of description, and shall not be understood as an indication or implication of relative importance or implicit indication of a quantity of indicated technical features. Therefore, a feature limited by "first" or "second" may explicitly or implicitly include at least one of the features.

In the descriptions of this application, "a plurality of" means at least two, for example, two or three, unless otherwise clearly limited.

In this application, unless otherwise clearly specified and limited, terms such as "installation", "connected", "connection", and "fastened" should be understood in a broad sense, for example, may be a fixed connection, or may be a detachable connection, or integration; or may be a direct connection, or may be an indirect connection through an intermediate medium, or may be internal communication of two elements or an interaction relationship between two elements, unless otherwise clearly limited. A person of ordinary skill in the art may understand specific meanings of the foregoing terms in this application based on a specific case.

In this application, unless otherwise clearly specified and limited, when a first feature is "above" or "below" a second feature, the first feature may be in direct contact with the second feature, or the first feature may be in indirect contact with the second feature through an intermediate medium. In addition, that the first feature is "above", "over", and "beyond" the second feature may be that the first feature is right above or obliquely above the second feature or merely indicate that the first feature is horizontally higher than the second feature. That the first feature is "below", "under", or "beneath" the second feature may be that the first feature is right below or obliquely below the second feature or merely indicate that the first feature is horizontally lower than the second feature.

In the descriptions of this application, it should be understood that the terms such as "inner", "outer", "side", "upper", "bottom", "front", and "back" indicate an orientation or a location relationship only for ease of describing this application and simplifying description, but do not indicate or imply that a specified apparatus or element needs to have a specific orientation and be constructed and operated in a specific orientation. Therefore, such terms cannot be understood as a limitation on this application.

In the descriptions of this application, it should be noted that the term "and/or" describes only an association relationship for describing associated objects and indicates that three relationships may exist. For example, A and/or B may indicate the following three cases: Only A exists, both A and B exist, and only B exists.

It should be further noted that a same reference numeral in embodiments of this application represents a same component part or a same part or component. For a same part or component in embodiments of this application, only one part or component may be marked with a reference numeral as an example in the figure. It should be understood that, for another same part or component, the reference numeral is also applicable.

With development of terminal devices such as a smart watch, a smart band, smart glasses, and a headset, more terminal devices are with a health status detection function, that is, a wearer may detect a body by using the health status detection function in the terminal device. For example, an ECG electrode may be disposed in the terminal device to obtain electrocardiograph data of the wearer, and detect a heart rate, atrial contraction, and contraction or diastole of a ventricle of the wearer; or a PPG module may be disposed in the terminal device, and the PPG module is used to measure a blood oxygen concentration of the wearer; or both an ECG electrode and a PPG module may be disposed in the terminal device, to measure blood pressure (Blood Pressure, BP) and the like of the wearer by combining the ECG electrode and the PPG module.

For example, the terminal device is a smartwatch, and blood pressure is measured by using the smartwatch. A principle of performing blood pressure measurement in a manner of combining the ECG electrode and the PPG module is as follows: First time of contraction of a blood vessel on a wrist of the wearer may be measured through the PPG module, second time of contraction of a ventricle of the wearer may be measured through direct or indirect contact between skin and the ECG electrode, and time of conducting a pulse wave from the ventricle to the wrist, namely, pulse transit time PTT, may be obtained based on the first time and the second time. Assuming that a distance between the ventricle and the wrist is L (during actual application, the distance may be obtained based on body parameter information such as a height and a weight of the wearer), a pulse wave velocity PWV may be obtained based on L and the PTT, where the PWV and the PTT meet the following formula (1):

$$PWV = \frac{L}{PTT} \tag{1}$$

According to the foregoing formula (1), when the distance between the ventricle and the wrist is fixed, shorter PTT time indicates a faster PWV; on the contrary, longer PTT time indicates a slower PWV.

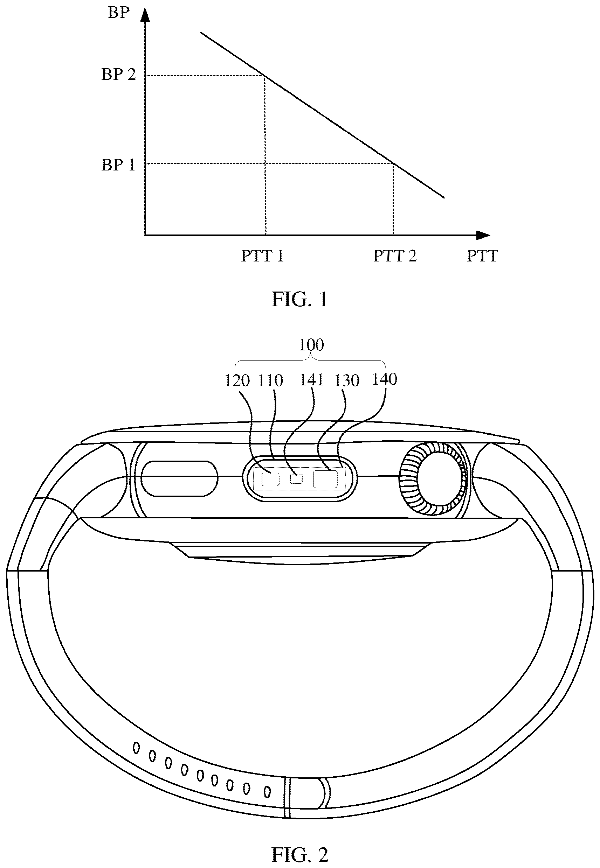

Based on a diagram of a relationship between the BP and the PTT shown in FIG. 1, it can be learned that there is a linear relationship between the PTT and the BP. Shorter PTT time indicates higher BP; on the contrary, longer PTT time indicates lower BP. That is, the PTT and the BP meet the following formula (2):

$$BP = a \times PTT + b \tag{2}$$

It is not difficult to understand that the PPG module includes a light-emitting diode (Light-Emitting Diode, LED) and a photosensitive diode (Photo Diode, PD). The LED (namely, an optical transmitter in embodiments of this application) irradiates an optical signal of a specific wavelength to a human body tissue and blood. After the optical signal is reflected by the human body tissue and the blood, the optical signal is received by the PD (namely, an optical receiver in embodiments of this application). The optical signal transmitted by the LED is compared with the optical signal received by the PD, so that a blood flow characteristic and data of the contraction of the blood vessel (including the first time of the contraction of the blood vessel herein) corresponding to the PPG module may be obtained. ECG records electrophysiological activities of a heart in a unit of time, and is usually displayed in a wave form (namely, a common electrocardiogram). When the skin of the wearer touches the ECG electrode, an overall potential change of the heart can be directly detected. The electrocardiogram includes a P wave, a QRS wave, and a T wave, where the P wave indicates an atrial contraction status, the QRS wave indicates a status of contraction of a ventricle, and the T wave indicates a status of diastole of the ventricle. The QRS wave may be further divided into a Q wave, an R wave, and an S wave. Interval time between two adjacent R waves may be used to measure a heart rate, and second time of the contraction of the ventricle may be obtained based on the R wave.

In actual structure disposition, the ECG electrode and the PPG module are usually separately disposed in the terminal device. As a result, a structure design of the ECG electrode and the PPG module occupies an excessively large proportion of space of the entire terminal device. This is not conducive to miniaturization development of the terminal device. In addition, in a manner in which the ECG electrode and the PPG module are separately disposed, when health detection such as detection of blood pressure is performed, some skin of the wearer needs to be in contact with the PPG module to collect a PPG signal (corresponding to the foregoing blood flow characteristic and the foregoing data of the contraction of the blood vessel corresponding to the PPG module), and some skin is electrically connected to the ECG electrode to collect an ECG signal (corresponding to the foregoing overall potential change of the heart). In other words, if the ECG electrode and the PPG module are separately disposed, blood pressure measurement can be performed only after the PPG signal and the ECG signal are separately collected, and a health detection process is complex.

Therefore, for a problem that the structure design of the ECG electrode and the PPG module in the existing terminal device occupies large space, FIG. 2 is a diagram of a structure of a side surface of a detection apparatus 100 disposed in a smartwatch according to an embodiment of this application. As shown in FIG. 2, an optical receiver 120, an optical transmitter 130, and a first electrode 141 are separately connected to a flexible printed circuit (Flexible Printed Circuit, FPC) 140 through a support component 110, to form the detection apparatus 100. When skin of a user is in contact with an outer surface of the detection apparatus 100, the optical receiver 120, the optical transmitter 130, the first electrode 141, and the like may be used to detect a health status such as blood pressure. This structure design avoids a problem that the large space is occupied because of the structure design in which the ECG electrode (corresponding to the first electrode 141 in the detection apparatus 100) and the PPG module are separated, and effectively resolves a problem that the structure design of the ECG electrode and the PPG module occupies an excessively large proportion of space of the entire device. In addition, in an actual use process, a corresponding PPG signal and a corresponding ECG signal can be collected by the optical receiver 120, the optical transmitter 130, and the first electrode 141 at the same time, provided that only one skin detection site is in contact with the outer surface of the detection apparatus 100, to complete detection of the blood pressure. This structure design makes a health detection operation simpler, and effectively improves product practicability.

The detection apparatus 100 provided in embodiments of this application may be used in an electronic device (also referred to as a terminal device). For example, assuming that the electronic device is a smartwatch or a band, as shown in FIG. 2, the electronic device includes a housing and the foregoing detection apparatus 100. The detection apparatus 100 is embedded on an outer surface of a side wall of the housing, and skin is in contact with the outer surface of the detection apparatus 100, so that health statuses such as a heart rate, blood oxygen saturation, an electrocardiogram, and blood pressure of a user can be detected.

Optionally, a second electrode is further embedded on a bottom wall of the housing, and the second electrode and the first electrode 141 in the detection apparatus 100 are configured to form a path to collect an electrical signal when the skin is separately in contact with a top end surface of the support component 110 in the detection apparatus 100 and the bottom wall of the housing. In other words, when the detection apparatus 100 in embodiments of this application is used in the electronic device shown in FIG. 2, after the user wears the smartwatch or the band on a wrist, the skin is electrically connected to the second electrode through contact between the wrist and the second electrode on the bottom wall of the housing, and then the skin is electrically connected to the first electrode 141 by placing a finger on the top end surface of the support component 110 in the detection apparatus 100. In this way, the first electrode 141 is electrically connected to the second electrode through the skin, and the first electrode 141 and the second electrode that are electrically connected may collect the electrical signal of the skin.

It should be understood that, after the electronic device is worn by the user, the second electrode disposed on the bottom wall of the housing may be in direct contact with skin of a wearing part, so that the skin is electrically connected to the second electrode. Therefore, in an actual design, the first electrode 141 and the second electrode may be used to collect the electrical signal of the skin provided that the skin is electrically connected to the first electrode 141 through the detection apparatus 100.

Optionally, the electronic device may further include a processor, and the FPC 140 in the detection apparatus 100 is electrically connected to the processor in the electronic device through a board-to-board BTB connector.

Optionally, a quantity of PINs of the BTB connector is 10. It should be understood that the quantity of PINs of the BTB connector is related to a quantity of health detection functions disposed in the detection apparatus 100.

Optionally, a disposition height of the detection apparatus 100 on the side wall of the housing may correspond to the outer surface of the side wall of the housing (for example, the disposition height is level with the outer surface); or the disposition height of the detection apparatus 100 on the side wall of the housing may be higher than the outer surface of the side wall of the housing, that is, the detection apparatus 100 is disposed protruding from the side wall of the housing. A specific disposition manner of the detection apparatus 100 on the electronic device is not limited in this application.

It is not difficult to understand that the electronic device may be a mobile phone, a tablet computer, a smart band, a smartwatch, a smart head-mounted display, smart glasses, an augmented reality (Augmented Reality, AR)/virtual reality (Virtual Reality, VR) device, a notebook computer, an ultra-mobile personal computer (Ultra-Mobile Personal Computer, UMPC), a netbook, a personal digital assistant (Personal Digital Assistant, PDA), a vehicle-mounted device, a smart screen, a cloud server, or the like. A specific type of the electronic device is not limited in embodiments of this application.

The following describes in detail the detection apparatus 100 provided in this application by using specific embodiments.

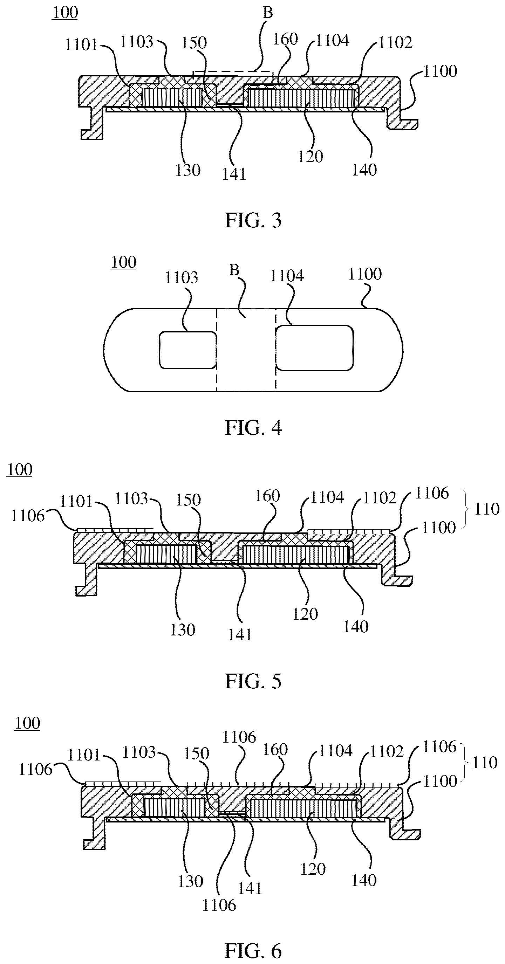

FIG. 3 is a diagram of a cross-sectional structure of a detection apparatus 100 according to an embodiment of this application. FIG. 4 is a diagram of a top-view structure of a detection apparatus 100 according to an embodiment of this application. As shown in FIG. 3 and FIG. 4, the detection apparatus 100 includes a support component 110 (refer to a support component body 1100 in FIG. 3, and the same applies to the following), an optical receiver 120, an optical transmitter 130, and an FPC 140. A first groove 1101 and a second groove 1102 are provided on a bottom end surface of the support component 110. The bottom end surface of the support component 110 is connected to the FPC 140. The first groove 1101 and the FPC 140 form a first accommodating cavity 150 configured to accommodate the optical transmitter 130, and the second groove 1102 and the FPC 140 form a second accommodating cavity 160 configured to accommodate the optical receiver 120. In other words, when the bottom end surface of the support component 110 covers the FPC 140, the first accommodating cavity 150 corresponding to the first groove 1101 and the second accommodating cavity 160 corresponding to the second groove 1102 are formed. A first light exit window 1103 and a first light entry window 1104 are provided on a top end surface of the support component 110. The first light exit window 1103 communicates with the first groove 1101, the first light entry window 1104 communicates with the second groove 1102, the first light exit window 1103 is used for emission of a light ray emitted by the optical transmitter 130, and the first light entry window 1104 is configured to receive a reflected light ray by the optical receiver 120.

Both the optical transmitter 130 and the optical receiver 120 are electrically connected to the FPC 140. The optical transmitter 130 is disposed in the first accommodating cavity 150, and the optical receiver 120 is disposed in the second accommodating cavity 160. A first electrode 141 is further disposed on the FPC 140, and the first electrode 141 is electrically connected to the support component 110.

In a possible implementation, the optical transmitter 130 is configured to transmit an optical signal, and the optical receiver 120 is configured to receive the reflected optical signal. For example, the optical transmitter 130 may be an LED, and the optical receiver 120 may be a PD.

In a possible implementation, the support component 110 may be integrally formed into an integrated structure. As shown in FIG. 3 and FIG. 4, specifically, the support component 110 includes the bottom end surface and the top end surface, the first groove 1101 used to accommodate the optical transmitter 130 and the second groove 1102 used to accommodate the optical receiver 120 are provided on the bottom end surface of the support component 110, and the first light exit window 1103 corresponding to the optical transmitter 130 and the first light entry window 1104 corresponding to the optical receiver 120 are provided on the top end surface of the support component 110. The first light exit window 1103 correspondingly communicates with the first groove 1101, and the first light entry window 1104 correspondingly communicates with the second groove 1102. The light ray emitted by the optical transmitter 130 is emitted through the first light exit window 1103 and irradiated into tissue and blood, and the light ray reflected by the tissue and the blood is emitted through the first light entry window 1104 and received by the optical receiver 120.

Refer to FIG. 3 and FIG. 4. Not only the optical transmitter 130 and the optical receiver 120 that are separately electrically connected to the FPC 140 are disposed on the FPC 140, but also the first electrode 141 is disposed on the FPC 140. The first electrode 141 may be electrically connected to the top end surface of the support component 110, or may be directly electrically connected to the support component 110.

It is not difficult to understand that a specific disposition location of the first electrode 141 on the FPC 140 is flexible. By way of an example but not a limitation, the first electrode 141 may be disposed on the FPC 140 between the optical transmitter 130 and the optical receiver 120. In another possible implementation, the first electrode 141 may alternatively be disposed on one side that is of the FPC 140 and that is close to the optical transmitter 130 and far away from the optical receiver 120, may be disposed on one side that is of the FPC 140 and that is far away from the optical transmitter 130 and close to the optical receiver 120; or the like. The specific disposition location of the first electrode 141 on the FPC 140 is not limited in this application.

In a possible implementation, the support component 110 may include a support component body 1100, and the support component body 1100 is made of a conductive light-shielding material. For example, the conductive light-shielding material may be a material such as iron, copper, aluminum, titanium alloy, liquid metal, or conductive ceramic.

When the support component body 1100 is made of a conductive light-shielding material, as shown in FIG. 3 and FIG. 4, the support component body 1100 may be directly electrically connected to the first electrode 141. When skin is in contact with a top end surface of the support component body 1100, the skin communicates with the first electrode 141 through the electrical connection between the support component body 1100 and the first electrode 141.

In the possible implementation, the entire support component body 1100 may be made of a conductive light-shielding material. It is not difficult to understand that light-shielding of the support component body 1100 can avoid crosstalk between an optical signal transmitted by the optical transmitter 130 and an optical signal received by the optical receiver 120. This effectively improves accuracy of data detected by the detection apparatus 100.

Optionally, when the entire support component body 1100 is made of a conductive light-shielding material, at least one conductive material may be further disposed on the top end surface of the support component body 1100 or on a surface of the support component body 1100, to minimize abrasion on the top end surface of the support component body 1100 caused by frequent contact between the skin and the top end surface of the support component body 1100. For example, the conductive material may be a conductive light-transmitting or light-shielding adhesive, a conductive paint, tin soldering, metal powder, or the like.

In an actual design, a first part of the support component body 1100 may alternatively be made of a conductive light-shielding material, and a second part is not made of a conductive light-shielding material. The first part corresponds to the disposition location of the first electrode 141, and the second part may be specifically made of a light-shielding insulation material or a light-transmitting material.

For example, if the first electrode 141 is located between the optical transmitter 130 and the optical receiver 120, the first part (refer to a region B in FIG. 3 and FIG. 4, and the same applies to the following) of the support component body 1100 may be made of a conductive light-shielding material, the second part includes one end that is of the support component body 1100 and that is close to the optical transmitter 130 and one end that is of the support component body 1100 and that is close to the optical receiver 120, and all the second part may be made of a light-shielding insulation material. In other words, a conductive light-shielding material is used between the first accommodating cavity 150 and the second accommodating cavity 160 on the support component body 1100, the first part, of the support component body 1100, that corresponds to the conductive light-shielding material is directly electrically connected to the first electrode 141, and one side that is of the support component body 1100 and that is close to the first accommodating cavity 150 and away from the second accommodating cavity 160 and one side that is of the support component body 1100 and that is away from the first accommodating cavity 150 and close to the second accommodating cavity 160 are made of a light-shielding insulation material. In this way, when the skin is in contact with the top end surface of the support component body 1100, the skin may be electrically connected to the first electrode 141 by using the conductive light-shielding material on the support component body 1100.

In another possible embodiment, to increase an effective conductive contact area between the skin and the top end surface of the support component body 1100, in the example in FIG. 3 or FIG. 4, a surface of the light-shielding insulation material on the top end surface of the support component body 1100 may be coated with a conductive material, and the conductive material may partially or completely cover the first part, so that an electrical connection is generated between the conductive material and the conductive light-shielding material of the first part; or the surface of the support component body 1100 or the top end surface of the support component body 1100 may be completely coated with a conductive material.

FIG. 5 is a diagram of a cross-sectional structure of a first conductive coating 1106 disposed on the top end surface of the support component body 1100 according to an embodiment of this application. Refer to FIG. 5. In an example in which a part of the support component body 1100 is made of a conductive light-shielding material and a part of the support component body 1100 is made of a light-shielding insulation material, the first conductive coating 1106 may be coated on a top end surface that is of the support component body 1100 and that is close to one end of the optical transmitter 130, and/or the first conductive coating 1106 may be coated on a top end surface that is of the support component body 1100 and that is close to one end of the optical receiver 120. The first conductive coating 1106 is disposed on the top end surface and communicates with the conductive light-shielding material of the first part.

Optionally, the first conductive coating 1106 may be any one of a conductive light-transmitting adhesive, a conductive light-shielding adhesive, a conductive paste, or a conductive paint.

In this example, the first conductive coating 1106 may also be coated on a top end surface of the conductive light-shielding material of the first part, so that the top end surface of the support component body 1100 is remained flat.

It should be understood that, for a related example in which the first part of the support component body 1100 is made of a conductive light-shielding material and the second part is made of a light-shielding insulation material, the first conductive coating 1106 disposed on the conductive light-shielding material or the light-shielding insulation material may be light-transmitting, or may be light-shielding. This facilitates selection of a material of the first conductive coating 1106 in an actual design, reduces design difficulty, and simplifies structure disposition. When the first conductive coating 1106 is light-transmitting, the first conductive coating 1106 coated on the top end surface of the support component body 1100 may cover the first light exit window 1103 and/or the first light entry window 1104. When the first conductive coating 1106 is light-shielding, the first conductive coating 1106 coated on the top end surface of the support component body 1100 needs to avoid the first light exit window 1103 and the first light entry window 1104, to avoid blocking transmission of the optical signal transmitted by the optical transmitter 130 and transmission of the optical signal received by the optical receiver 120 due to coverage of the light-shielding first conductive coating 1106.

It is not difficult to understand that, in another possible implementation, specific types of the first conductive coating 1106 disposed on the top end surface of the support component body 1100 (including the conductive light-shielding material of the first part, the top end surface that is of the support component body 1100 and that is close to the end of the optical transmitter 130, and the top end surface that is of the support component body 1100 and that is close to the end of the optical receiver 120) may be completely the same, partially the same, or may be completely different. This is not limited in this application.

It should be noted that, based on the foregoing possible example, a disposition thickness and a coating area of the first conductive coating 1106 on the top end surface of the support component body 1100 and coverage proportions of different specific types of the first conductive coating 1106 on the surface or the top end surface of the support component body 1100 all may be set based on an actual application requirement (for example, a quantity of materials of the first conductive coating 1106). This is not limited in this application.

In addition to the foregoing possible example, as shown in FIG. 5, when the first part of the support component body 1100 is made of a conductive light-shielding material, and the end that is of the support component body 1100 and that is close to the optical transmitter 130 and the end that is of the support component body 1100 and that is close to the optical receiver 120 are made of a light-shielding insulation material, a conductive light-shielding layer may also be coated on the top end surface that is of the support component body 1100 and that is close to the end of the optical transmitter 130, and/or a conductive light-shielding layer may be coated on the top end surface that is of the support component body 1100 and that is close to the end of the optical receiver 120. The conductive light-shielding layer is disposed on the top end surface and needs to communicate with the conductive light-shielding material of the first part, and the conductive light-shielding layer is disposed on the top end surface and needs to avoid the first light exit window 1103 and the first light entry window 1104.

Based on this example, the conductive light-shielding layer may include any one of a silkscreen, conductive ink, a conductive adhesive, a black matrix, or a light-shielding sheet.

Similarly, the conductive light-shielding layer may also be coated on the top end surface of the conductive light-shielding material of the first part.

It is not difficult to understand that, in this example, a disposition thickness and a coverage area of the conductive light-shielding layer on the top end surface of the support component body 1100 may be specifically designed based on an actual design size of the detection apparatus 100. This is not limited in this application.

Optionally, when a part of the support component body 1100 is made of a conductive light-shielding material, and a part of the support component body 1100 is made of a light-shielding insulation material, both the first conductive coating 1106 and the conductive light-shielding layer may be disposed on the top end surface of the support component body 1100 or the surface of the support component body 1100. Specific types of the first conductive coating 1106 and the conductive light-shielding layer that are disposed on the top end surface of the support component body 1100 or on the surface of the support component body 1100, a disposition area, a specific disposition location, and the like of each specific type of conductive material are not limited in this application.

In another possible implementation, in the foregoing embodiment in which the support component body 1100 is made of a conductive light-shielding material, the first part of the support component body 1100 may be made of a conductive light-shielding material, and the end that is of the support component body 1100 and that is close to the optical transmitter 130 and the end that is of the support component body 1100 and that is close to the optical receiver 120 may also be made of a light-transmitting material in addition to a light-shielding insulation material.

When the first part of the support component body 1100 is made of a conductive light-shielding material, and the end that is of the support component body 1100 and that is close to the optical transmitter 130 and the end that is of the support component body 1100 and that is close to the optical receiver 120 are made of a light-transmitting material, at least one coating (for example, the conductive light-shielding layer) that is both light-shielding and conductive may be disposed on the surface that is of the support component body 1100 and that is made of a light-transmitting material. For a specific disposition manner of the coating that is both light-shielding and conductive, refer to a specific disposition manner of the conductive light-shielding layer in the embodiment in which a part of the support component body 1100 is made of a conductive light-shielding material and a part of the support component body 1100 is made of a light-shielding insulation material. Details are not described herein again.

It should be understood that the foregoing embodiment merely describes an example in which the support component body 1100 is of an integrated structure, the first electrode 141 is disposed between the optical transmitter 130 and the optical receiver 120, and a part of the support component body 1100 is made of a conductive light-shielding material and a part of the support component body 1100 is not made of a conductive light-shielding material. In an actual processing and production process, related structure design details may change as a disposition location of the first electrode 141 on the FPC 140 changes. This is not limited in this application.

Based on the foregoing example, to strengthen firmness of the connection between the support component body 1100 and the first electrode 141 and enhance conductivity between the support component body 1100 and the first electrode 141, optionally, the first conductive coating 1106 may be disposed on a contact surface between the support component body 1100 and the first electrode 141, or the conductive light-shielding layer may be disposed on the contact surface between the support component body 1100 and the first electrode 141. In another possible example, another material that has both a bonding function and conductivity may be disposed on the contact surface between the support component body 1100 and the first electrode 141. A specific type of the another material is not limited in this application.

It should be noted that when the specific disposition location of the first electrode 141 on the FPC 140 is located between the optical transmitter 130 and the optical receiver 120, a conductive material (for example, a conductive light-shielding paint) for shielding light may be selected for the first conductive coating 1106 disposed on the contact surface between the support component body 1100 and the first electrode 141, to better isolate a light ray emitted by the optical transmitter 130 from a light ray received by the optical receiver 120, thereby avoiding a case in which a health monitoring parameter is inaccurate due to cross of the light rays. Certainly, when a thickness of the conductive material is thin (that is, light transmitting of the conductive material does not affect normal use of the detection apparatus 100), light transmitting of the first conductive coating 1106 may not be considered.

Based on the support component body 1100 made of a conductive light-shielding material, in a possible implementation, as shown in FIG. 3 to FIG. 5, the first light exit window 1103 disposed on the support component body 1100 may be a second through hole provided on the support component body 1100, and/or the first light entry window 1104 may be a third through hole provided on the support component body 1100. In this design manner, a structure is simple and is easy to implement, and this facilitates visual installation between the support component body 1100 and the FPC 140.

Based on an actual application requirement, the detection apparatus 100 provided in this embodiment of this application is usually installed in an electronic device. To improve waterproof performance, mould-proof performance, and dust-proof performance of the detection apparatus 100, improve product practicability, further protect the optical transmitter 130 in the first accommodating cavity 150 and the optical receiver 120 in the second accommodating cavity 160, and prevent a light ray emitted by the optical transmitter 130 from passing through the first light exit window 1103 and a light ray received by the optical receiver 120 from passing through the first light entry window 1104, in this embodiment of this application, the first light exit window 1103 may be a second through hole provided on the support component body 1100, and a first transparent part is disposed in the second through hole; and/or the first light entry window 1104 may be a third through hole provided on the support component body 1100, and a second transparent part is disposed in the third through hole.

The first transparent part and/or the second transparent part may be any one of a transparent adhesive, transparent plastic, or transparent silicone. A specific type of the first transparent part disposed in the second through hole may be the same as or different from a specific type of the second transparent part disposed in the third through hole.

To effectively ensure accuracy of health measurement performed through the optical transmitter 130 and the optical receiver 120, in a possible implementation, transmittance of the first light exit window 1103 and/or transmittance of the first light entry window 1104 may be greater than 90%. In other words, a material of the first light exit window 1103 and/or the first light entry window 1104 may be a material whose transmittance is greater than 90%. The material whose transmittance is greater than 90% may be glass, air, silicone, or the like.

In another possible implementation, the support component 110 includes the support component body 1100 and a conductive material disposed on a surface of the support component body 1100.

In this implementation, the support component body 1100 may be made of a light-shielding insulation material (namely, a light-shielding insulation material). For example, the light-shielding insulation material may be black plastic, light-shielding ceramic, light-shielding rubber, blackened glass, or the like.

Refer to FIG. 6. When the support component body 1100 is made of a light-shielding insulation material, a first conductive coating 1106 may be disposed on the surface of the support component body 1100, and the first electrode 141 may be directly electrically connected to the first conductive coating 1106 disposed on the surface of the support component body 1100. When the skin is in contact with the first conductive coating 1106 on the top end surface of the support component body 1100, the skin may be electrically connected to the first electrode 141 through the electrical connection between the first conductive coating 1106 and the first electrode 141.

It should be noted that, during actual disposition, when the support component body 1100 is made of a light-shielding insulation material, the first conductive coating 1106 coated on the surface of the support component body 1100 may be specifically disposed on the top end surface, a bottom end surface, and a side end surface of the support component body 1100, that is, the first conductive coating 1106 is disposed around an entire outer surface of the support component body 1100. Such disposition is to implement, when the support component body 1100 is made of a light-shielding insulation material, the electrical connection between the skin and the first electrode 141 by using the first conductive coating 1106 disposed on an entire outer surface of the support component 110.

In FIG. 6, for light transmitting of the first conductive coating 1106 disposed on the surface of the support component body 1100 and a specific disposition manner of the first conductive coating 1106 in the first light exit window 1103 and/or the first light entry window 1104, refer to related descriptions of the light transmitting of the first conductive coating 1106 and a specific disposition status of the first conductive coating 1106 in the first light exit window 1103 and/or the first light entry window 1104 in the embodiment in which the first part of the support component 110 is made of a conductive light-shielding material and the second part of the support component 110 is made of a light-shielding insulation material. Details are not described herein again.

In another possible implementation, when the support component body is made of a light-shielding insulation material, the first conductive coating 1106 disposed on the surface of the support component body 1100 may alternatively be a conductive light-shielding layer. In this way, when the skin is in contact with the conductive light-shielding layer on the top end surface of the support component body 1100, the electrical connection between the skin and the first electrode 141 can be implemented by disposing the conductive light-shielding layer on the surface of the support component body 1100. For a specific disposition manner of disposing the conductive light-shielding layer on the surface of the support component body 1100, refer to the specific disposition manner of the conductive light-shielding layer in the embodiment in which the first part of the support component 110 is made of a conductive light-shielding material and the second part of the support component 110 is made of a light-shielding insulation material. Details are not described herein again.

In addition to the foregoing two possible implementations, when the support component body 1100 is made of a light-shielding insulation material, another conductive material that is light-transmitting or light-shielding may also be disposed on the surface of the support component body 1100. When the another conductive material is light-shielding, it needs to be noted that the another conductive material that is light-shielding is coated to avoid the first light entry window 1104 and the first light exit window 1103.

It should be noted that, for a case in which the support component body 1100 is made of a light-shielding insulation material, refer to related descriptions in the embodiment in which the support component body 1100 is made of a conductive light-shielding material. A difference is that when the support component body 1100 is light-shielding and conductive, the conductive light-shielding material in the support component body 1100 may be electrically connected to the first electrode 141. Therefore, a specific disposition location of the conductive material coated on the support component body 1100 is mainly on the top end surface of the support component body 1100. In this embodiment, when the support component body 1100 is made of a light-shielding insulation material, at least one conductive material (or coating) needs to be disposed on the surface of the support component body 1100, so that the first electrode 141 establishes an electrical connection path to the top end surface of the support component 110 by using the conductive coating.

In another possible implementation, the support component body 1100 may alternatively be made of a light-transmitting material. The light-transmitting material may be specifically classified into a conductive light-transmitting material and a light-transmitting insulation material (namely, a light-transmitting insulation material). For example, the conductive light-transmitting material may be indium tin oxide or the like, and the light-transmitting insulation material may be transparent glass, transparent plastic, transparent silicone, rubber, or the like. Based on the light-transmitting material of the support component body 1100, the support component 110 may include the support component body 1100 and a material that is disposed on the surface of the support component body 1100 and that is both light-shielding and conductive.

For a case in which the support component body 1100 is made of a light-transmitting material, refer to FIG. 6. The first conductive coating 1106 that is light-shielding is coated on the surface of the support component body 1100, so that when the skin is in contact with the top end surface of the support component 110, the electrical connection between the skin and the first electrode 141 can be implemented by using the first conductive coating 1106 that is light-shielding.

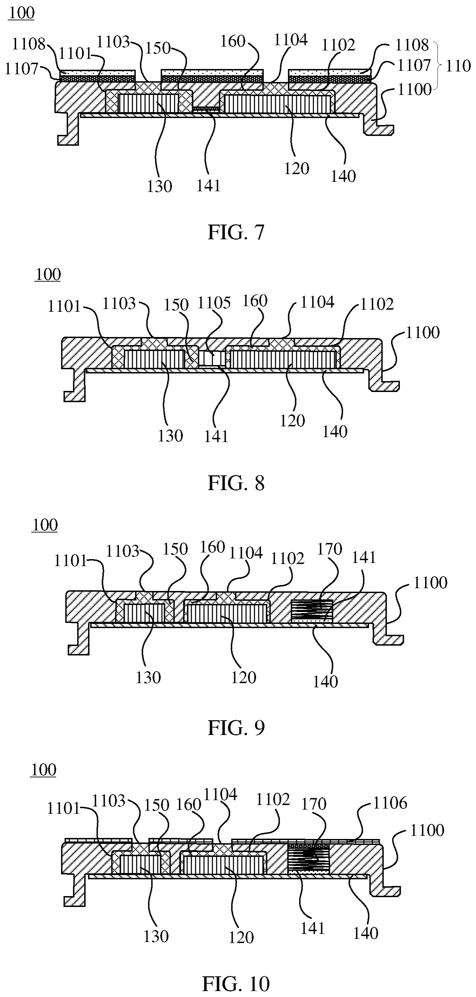

To block transmission of a light ray in a region other than the first light exit window 1103 and the first light entry window 1104, and improve utilization of the light ray, in this embodiment of this application, if the support component body 1100 is made of a light-transmitting material, refer to FIG. 8, so that a conductive light-shielding layer can be disposed on the surface of the support component body 1100. The first electrode 141 is directly connected to the conductive light-shielding layer on the surface of the support component body 1100. In this way, after the skin is in contact with the conductive light-shielding layer, the electrical connection between the skin and the first electrode 141 can be implemented.

In another possible example, if the support component body 1100 is made of a light-transmitting material, further refer to FIG. 7, so that the surface of the support component body 1100 may be covered with a light-shielding insulation layer 1107, the light-shielding insulation layer 1107 is covered with a second conductive coating 1108, and the first electrode 141 communicates with the second conductive coating 1108 on the surface of the support component body 1100. In this way, after the skin is in contact with the second conductive coating 1108 on the surface of the support component body 1100, the skin may be electrically connected to the first electrode 141.

In other words, when the support component body 1100 is made of a light-transmitting material, not only light-shielding processing (or blackening processing) needs to be performed on the support component body 1100 made of the light-transmitting material, but also conductivity of the support component 110 needs to be considered using the support component 110 as a body or a support body that is electrically connected to the first electrode 141. Therefore, the surface of the support component body 1100 may be directly coated with the conductive light-shielding layer, or the surface of the support component body 1100 may be coated with the light-shielding insulation layer 1107, and then a surface of the light-shielding insulation layer 1107 is coated with the second conductive coating 1108, so that the support component 110 has conductivity and can be directly connected to the first electrode 141, the support component 110 is light-shielding, and the optical transmitter 130 and the optical receiver 120 disposed in the support component 110 can work efficiently.

Optionally, the light-shielding insulation layer 1107 may be a silicone layer or a rubber layer to which a dye is added, a coating sprayed with extinction ink or black ink, glass and plastic obtained after blackening processing, or the like.

In this implementation, for specific disposition manners of the first light exit window 1103 and the first light entry window 1104 disposed on the support component 110, refer to the design manners in the embodiment in which the support component 110 is made of a conductive light-shielding material. In addition, when the support component body 1100 is made of a light-transmitting material, a material of the first light exit window 1103 provided on the support component 110 may be the same as a material of the support component body 1100, and/or a material of the first light entry window 1104 provided on the support component 110 may also be the same as the material of the support component body 1100.

In other words, the first light exit window 1103 is a region, on the support component body 1100, that is not coated with the first conductive coating 1106 that is light-shielding, the conductive light-shielding layer, the light-shielding insulation layer 1107, and the second conductive coating 1108. The first light entry window 1104 is a region, on the support component body 1100, that is not coated with the first conductive coating 1106 that is light-shielding, the conductive light-shielding layer, the light-shielding insulation layer 1107, and the second conductive coating 1108. In this case, the support component body 1100, the first light exit window 1103, and the first light entry window 1104 may be considered as a whole, that is, the first light exit window 1103 and the first light entry window 1104 belong to a part of the support component body 1100.

Optionally, when the support component body 1100 is made of a light-transmitting material, the second conductive coating 1108 disposed on the light-shielding insulation layer 1107 may be light-transmitting, or may be light-shielding. In this example, for light transmitting of the second conductive coating 1108 disposed on the light-shielding insulation layer 1107 and a specific disposition manner of the second conductive coating 1108 in the first light exit window 1103 and/or the first light entry window 1104, refer to related descriptions of the light transmitting of the first conductive coating 1106 and a specific disposition status of the first conductive coating 1106 in the first light exit window 1103 and/or the first light entry window 1104 in the embodiment in which the first part of the support component 110 is made of a conductive light-shielding material and the second part of the support component 110 is made of a light-shielding insulation material. Details are not described herein again.

In the foregoing possible example, when the material of the support component body 1100 is made of a light-transmitting material, a plurality of groups of stacked light-shielding insulation layers 1107 and second conductive coatings 1108 may alternatively be sequentially disposed on the surface of the support component body 1100; or a plurality of light-shielding insulation layers 1107 are disposed on the surface of the support component body 1100, and then a second conductive coating 1108 is disposed on a surface of an outermost light-shielding insulation layer 1107. A quantity of disposed layers and a disposition thickness of the conductive light-shielding layer or the light-shielding insulation layer 1107 disposed on the surface of the support component body 1100, and a quantity of groups of the light-shielding insulation layer 1107 and the second conductive coating 1108 that are repeatedly disposed on the surface of the support component body 1100 are not limited in this application.

It should be noted that, to clearly see the first conductive coating 1106 that is light-shielding, the conductive light-shielding layer, the light-shielding insulation layer 1107, and the second conductive coating 1108 that are disposed on the support component body 1100, refer to FIG. 6 and FIG. 7. In this embodiment of this application, the first conductive coating 1106 that is light-shielding, the conductive light-shielding layer, the light-shielding insulation layer 1107, and the second conductive coating 1108 are all disposed with a specific thickness. In an actual production and manufacturing process, the foregoing coatings with different thicknesses may be disposed based on different materials of the support component body 1100 in the actual detection apparatus 100. This is merely an example in this application.

Regardless of whether the support component body 1100 is made of a conductive light-shielding material, a light-shielding insulation material, a conductive light-transmitting material, or a light-transmitting insulation material, in a possible implementation, a volume of the first accommodating cavity 150 formed by connecting the bottom end surface of the support component 110 to the FPC 140 may correspond to a volume of the optical transmitter 130, that is, an outer surface of the optical transmitter 130 is closely attached to a side wall of the first accommodating cavity 150; or the volume of the first accommodating cavity 150 may be greater than the volume of the optical transmitter 130, that is, the outer surface of the optical transmitter 130 is not attached to the side wall of the first accommodating cavity 150.

Similarly, a shape of an inner side wall of the first accommodating cavity 150 may be the same as a shape of an outer side wall of the optical transmitter 130. For example, the outer side wall of the optical transmitter 130 is a cylinder, and the inner side wall of the first accommodating cavity 150 may also be a cylinder. Certainly, the shape of the inner side wall of the first accommodating cavity 150 may be different from the shape of the outer side wall of the optical transmitter 130. For example, the outer side wall of the optical transmitter 130 is a cuboid, and the inner side wall of the first accommodating cavity 150 is a square.

Similarly, a volume of the second accommodating cavity 160 formed by connecting the bottom end surface of the support component 110 to the FPC 140 may correspond to a volume of the optical receiver 120, or may be greater than the volume of the optical receiver 120. A shape of an inner side wall of the second accommodating cavity 160 may be the same as a shape of an outer side wall of the optical receiver 120, or may be different from the shape of the outer side wall of the optical receiver 120.

To further improve waterproof performance and mould-proof performance of the optical transmitter 130 and/or the optical receiver 120, a surface of the optical transmitter 130 may be covered with a first transparent part, and/or a surface of the optical receiver 120 may be covered with a second transparent part. The first transparent part may be disposed around the surface of the optical transmitter 130, and fill the first accommodating cavity 150; and/or the second transparent part may be disposed around the surface of the optical receiver 120, and fill the second accommodating cavity 160. The first transparent part and/or the second transparent part may be any one of a transparent adhesive, transparent plastic, or transparent silicone.

It should be understood that a specific type of the first transparent part covering on the surface of the optical transmitter 130 may be the same as or different from a specific type of the second transparent part covering on the surface of the optical receiver 120. For example, both the first transparent part covering the surface of the optical transmitter 130 and the second transparent part covering the surface of the optical receiver 120 are a transparent adhesive; or the first transparent part covering the optical transmitter 130 is a transparent adhesive, and the second transparent part covering the optical receiver 120 is transparent plastic.

It should be noted that a specific type of the transparent part (refer to the first transparent part and the second transparent part) disposed on the surface of the optical transmitter 130 and/or the optical receiver 120 may be the same as or different from a specific type of the first transparent part disposed in the first light exit window 1103 or the second transparent part disposed in the first light entry window 1104.

It is not difficult to understand that, in this implementation, the volumes and/or the shapes of the first accommodating cavity 150 and the second accommodating cavity 160, whether the surface of the optical transmitter 130 and/or the surface of the optical receiver 120 are/is covered with the first transparent part and/or the second transparent part, a covering thickness of the first transparent part and/or a covering thickness of the second transparent part, and whether the specific type of the first transparent part and/or the specific type of the second transparent part are/is the same may be set based on an actual application requirement. This is not limited in this application.

In addition, a manner of the electrical connection between the support component 110 and the first electrode 141 may be direct rigid contact. In another possible implementation, FIG. 8 is a diagram of a structure of the first electrode 141 and the support component 110 that are electrically connected through a conductive portion 1105 according to an embodiment of this application. As shown in FIG. 8, the conductive portion 1105 is extended and disposed on the support component 110, and the support component 110 is electrically connected to the first electrode 141 through the conductive portion 1105.

It should be understood that FIG. 8 is merely an example of a structure of the detection apparatus 100 shown in FIG. 3 for description. A specific structure of the support component 110 and a specific structure design between the support component 110 and the FPC 140 in this possible implementation are the same as a specific structure of the support component body 1100 and a specific structure design between the support component body 1100 and the FPC 140 in the embodiment shown in FIG. 3. A difference is merely the manner of the electrical connection between the support component 110 and the first electrode 141. Same parts are not described herein again.

Specifically, as shown in FIG. 8, the conductive portion 1105 is extended and disposed on the bottom end surface of the support component 110, the conductive portion 1105 is located between a first groove 1101 and a second groove 1102, and the first electrode 141 corresponding to the conductive portion 1105 is disposed on the FPC 140. In this way, the first electrode 141 can be electrically connected to the support component 110 through the conductive portion 1105. Based on this implementation, the electrical connection between the support component 110 and the first electrode 141 is directly implemented by the conductive portion 1105 disposed on the support component 110. This helps simplify a structure design of the electrical connection between the support component 110 and the first electrode 141. In addition, the conductive portion 1105 may be disposed at a plurality of locations on the support component 110, and manufacturing is simple and convenient.

It should be understood that a material of the conductive portion 1105 may be any material of the support component body 1100 in the embodiment in which the support component 110 is directly electrically connected to the first electrode 141. In this implementation, for the material of the conductive portion 1105, disposition manners of various materials on the conductive portion 1105, and the like, refer to related descriptions of a specific material and a specific disposition manner of the support component body 1100 in the foregoing embodiment. Details are not described herein again.

It should be noted that, as shown in FIG. 8, a disposition width of the conductive portion 1105 corresponds to a disposition width of the first electrode 141. In this actual design, a specific structure of the conductive portion 1105 may also be different from the structure shown in FIG. 8. For example, the disposition width of the conductive portion 1105 may be greater than or less than the width of the first electrode 141, to increase or reduce a contact area between the conductive portion 1105 and the first electrode 141.

It is not difficult to understand that when a disposition location of the conductive portion 1105 in the support component 110 does not affect normal working of the optical transmitter 130 and the optical receiver 120, the material of the conductive portion 1105 may be any material that can electrically connect the support component 110 to the first electrode 141, and the specific structure design of the conductive portion 1105 may have a plurality of different cases, which greatly improves design flexibility of the conductive portion 1105. Therefore, the disposition location and the disposition structure of the conductive portion 1105 disposed on the support component 110 are not limited in this application, provided that when skin is in contact with the top end surface of the support component 110, a condition that the first electrode 141 can be electrically connected to the skin through the support component 110 and the conductive portion 1105 is met.

It should be noted that a function of disposing, on the support component 110, the conductive portion 1105 electrically connected to the first electrode 141 is not only to simplify the structure design of the support component 110 through the conductive portion 1105 whose disposition location is flexible, to simplify the structure design of the electrical connection between the support component 110 and the first electrode 141, but also to connect the support component 110 to the first electrode 141 by using more abundant materials or structures.

In another possible implementation, FIG. 9 is a diagram of a cross-sectional structure of the support component 110 and the first electrode 141 that are electrically connected through an elastic component 170 according to an embodiment of this application. Refer to FIG. 9. A third accommodating cavity configured to accommodate the elastic component 170 is provided on the support component 110, and the top end surface of the support component 110 is electrically connected to the first electrode 141 through the elastic component 170 disposed in the third accommodating cavity.

It should be understood that FIG. 9 is merely an example of a structure of the detection apparatus 100 shown in FIG. 3 for description. A specific structure of the support component 110 and a specific structure design between the support component 110 and the FPC 140 in this possible implementation are the same as a specific structure of the support component body 1100 and a specific structure design between the support component body 1100 and the FPC 140 in the embodiment shown in FIG. 3. A difference is merely the manner of the electrical connection between the support component 110 and the first electrode 141. Therefore, same parts are not described herein again.

It should be understood that the elastic component 170 may be any one of a spring plate, a spring, or a conducting wire. In this embodiment of this application, the elastic component 170 is a spring plate. As a conductive component that is electrically connected, the spring plate is configured to electrically connect the support component 110 to the first electrode 141. Such disposition can effectively ensure tightness of the electrical connection between the support component 110 and the first electrode 141.

In a possible implementation, the third accommodating cavity may be a groove provided on the support component 110. For example, refer to FIG. 9. A groove is provided on the bottom end surface of the support component 110. The groove is located on one side that is of the support component 110 and that is away from the first groove 1101 and close to the second groove 1102. The first electrode 141 is disposed on the FPC 140 corresponding to the groove. One end of the elastic component 170 is electrically connected to the bottom of the groove, and the other end of the elastic component 170 is electrically connected to the first electrode 141. When the skin is in contact with the top end surface of the support component 110, an electrical connection path is formed by the support component 110, the elastic component 170, and the first electrode 141, so that the electrical connection between the skin and the first electrode 141 is implemented.

It is not difficult to understand that, in the foregoing possible implementation, to implement that when the skin is in contact with the top end surface of the support component 110, the first electrode 141 can be effectively electrically connected to the skin through the elastic component 170 and the support component 110, an overall material of the support component 110 may be made of a conductive light-shielding material; or a partial region that is of the support component 110 and that is in contact with the elastic component 170 (one end that is of the support component 110 and that is close to the second groove 1102 and away from the first groove 1101) is made of a conductive material, and a material of the support component 110 in another region is made of another material. The another material includes a light-transmitting material (including a conductive light-transmitting material and a light-transmitting insulation material) and a light-shielding insulation material.

For a case in which the support component 110 is made of a conductive light-shielding material, as shown in FIG. 9, because the support component 110 is made of a conductive light-shielding material, one end of the elastic component 170 may be directly electrically connected to the bottom of the groove on the support component 110, and the other end of the elastic component 170 is electrically connected to the first electrode 141.

For a case in which the partial region that is of the support component 110 and that is in contact with the elastic component 170 is made of a conductive material, and the another region of the support component 110 is made of another material, a conductive coating may be disposed on the top end surface that is of the support component 110 and that is made of a light-shielding insulation material, and the conductive coating communicates with the conductive material in the partial region. In this way, when the skin is in contact with the top end surface of the support component 110, the conductive coating is electrically connected to the conductive material, and the first electrode 141 may be electrically connected to the skin through the elastic component 170, the conductive material, and the conductive coating. Certainly, in another optional manner, a conductive coating may alternatively be disposed on the entire top end surface of the support component 110.

It is not difficult to understand that in the foregoing possible implementation, for a specific disposition manner of the conductive coating on the support component 110, light transmitting of the conductive coating, disposition of a specific material of the conductive coating, and the like, refer to design manners of different conductive materials in the foregoing implementation in which the support component 110 is directly electrically connected to the first electrode 141. Details are not described herein again.

In a possible implementation, the third accommodating cavity may alternatively be a through hole provided on the support component 110. For example, refer to FIG. 10. A conductive support structure may be disposed at one end that is of the through hole and that is close to the top end surface of the support component 110. In an aspect, the conductive support structure is used for the electrical connection between the elastic component 170 and the top end surface of the support component 110. In another aspect, the conductive support structure provides a connection point for the elastic component 170 disposed in the through hole and provides support for the conductive material disposed on the top end surface of the support component 110. It should be understood that FIG. 10 is merely an example of the structure of the detection apparatus 100 shown in FIG. 3.

It should be understood that, based on the foregoing example, when the support component body 1100 is made of a light-shielding material (including a conductive light-shielding material or a light-shielding insulation material), the first conductive coating 1106 that is light-transmitting or light-shielding may be disposed on the top end surface of the support component body 1100 and a surface of the conductive support structure, as shown in FIG. 10; or the conductive light-shielding layer may be disposed on the top end surface of the support component body 1100 and the surface of the conductive support structure.

When the support component body 1100 is made of a light-transmitting material (including a conductive light-transmitting material or a light-transmitting insulation material), the first conductive coating 1106 that is light-shielding may be disposed on the top end surface of the support component body 1100 and the surface of the conductive support structure, as shown in FIG. 10; or the conductive light-shielding layer may be disposed on the top end surface of the support component body 1100 and the surface of the conductive support structure.

It can be understood that, in a disposition manner of the third accommodating cavity being a through hole, for a specific disposition manner of the first conductive coating 1106 on the support component body 1100, a specific disposition manner of the conductive light-shielding layer on the support component body 1100, light transmitting of the first conductive coating 1106, and the like, refer to explanations and descriptions in the foregoing related embodiment in which the support component body 1100 is made of a conductive light-shielding material. Details are not described herein again.

Optionally, a conductive material may be further disposed on a contact surface between the elastic component 170 and the support component body 1100; and/or a conductive material may be further disposed on a contact surface between the elastic component 170 and the first electrode 141.

For example, a manner in which the elastic component 170 is separately electrically connected to the support component body 1100 and the first electrode 141 may be: directly bonding two ends of the elastic component 170 to the support component body 1100 and the first electrode 141 respectively through a conductive adhesive; or soldering two ends of the elastic component 170 between the support component body 1100 and the first electrode 141; or connecting one end of the elastic component 170 to the support component body 1100 through a conductive adhesive, and connecting the other end of the elastic component 170 to the first electrode 141 on the FPC 140 through tin soldering; or connecting one end of the elastic component 170 to the first electrode 141 on the FPC 140 through tin soldering, and implementing the electrical connection through direct rigid contact between the other end of the elastic component 170 and the support component body 1100.

It should be noted that, in the foregoing example, only an example in which the third accommodating cavity is provided on one side that is of the support component 110 and that is away from the first groove 1101 and close to the second groove 1102 is used for description. In an actual structure design, the third accommodating cavity provided on the support component 110 may alternatively be provided between the first groove 1101 and the second groove 1102, or the third accommodating cavity is provided on one side that is of the support component 110 and that is close to the first groove 1101 and away from the second groove 1102. It can be understood that, as a specific disposition location of the third accommodating cavity changes, disposition locations of the elastic component 170 in the third accommodating cavity and the first electrode 141 on the FPC 140 also change accordingly. The specific disposition location of the third accommodating cavity, the disposition location of the elastic component 170, and the disposition location of the first electrode 141 are not specifically limited in this application.

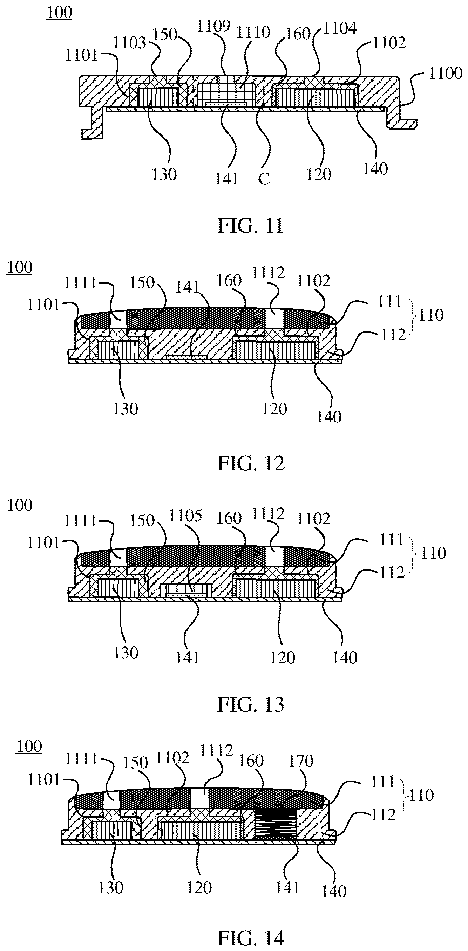

In another possible implementation, FIG. 11 is a diagram of a cross-sectional structure of the support component 110 and the first electrode 141 that are electrically connected by injecting a first conductive adhesive 1110 into the first through hole 1109 according to an embodiment of this application. Refer to FIG. 11. The first through hole 1109 corresponding to the first electrode 141 is provided on the support component 110, the first conductive adhesive 1110 is disposed in the first through hole 1109, and the top end surface of the support component 110 and/or the support component 110 are/is electrically connected to the first electrode 141 through the first conductive adhesive 1110.

It should be understood that FIG. 11 is merely an example of the structure of the detection apparatus 100 shown in FIG. 3. Specifically, as shown in FIG. 11, the first through hole 1109 is provided between the first groove 1101 and the second groove 1102 on the support component body 1100 (namely, the support component 110), the first conductive adhesive 1110 is disposed in the first through hole 1109, and the first conductive adhesive 1110 is disposed wrapping the first electrode 141, so that the first conductive adhesive 1110 is electrically connected to the first electrode 141; and a disposition height of the first conductive adhesive 1110 may be consistent with a depth of the first through hole 1109, so that the first conductive adhesive 1110 is electrically connected to the top end surface of the support component 110.

It should be understood that when the support component body 1100 is made of a conductive light-shielding material, the disposition height of the first conductive adhesive 1110 in the first through hole 1109 may be set randomly, provided that when the skin is in contact with the top end surface of the support component 110, the first electrode 141 can implement the electrical connection between the skin and the first electrode 141 by using conductivity between the first conductive adhesive 1110 and the support component 110.

For example, the disposition height of the first conductive adhesive 1110 in the first through hole 1109 is level with a height of the first electrode 141 relative to the FPC 140, and the first conductive adhesive 1110 is in contact with an inner side wall of the first through hole 1109. In this way, when the support component body 1100 is made of a conductive light-shielding material, the top end surface of the support component 110 can communicate with the first conductive adhesive 1110.

Based on this example, to prevent a foreign matter such as dust from falling into the first through hole 1109, a filler such as transparent plastic may be further laid on a top end surface of the first conductive adhesive 1110 in the first through hole 1109. For another example, the disposition height of the first conductive adhesive 1110 in the first through hole 1109 may alternatively be half of the depth of the first through hole 1109; or the disposition height of the first conductive adhesive 1110 in the first through hole 1109 may be the same as the depth of the first through hole 1109.

When a material of a partial region (for example, for a region around the first through hole 1109 on the support component 110, refer to a region C in FIG. 11) on the support component body 1100 is made of a conductive light-shielding material, and a material of another region is made of an insulation material, a conductive material may be disposed on the top end surface of the support component 110, to communicate with the conductive light-shielding material in the partial region through the conductive material. In this way, the disposition height of the first conductive adhesive 1110 in the first through hole 1109 may also be flexibly disposed. For details, refer to disposition of the first conductive adhesive 1110 when the support component 110 is made of a conductive light-shielding material. Details are not described herein again.

When the support component 110 is made of a light-shielding insulation material, the disposition height of the first conductive adhesive 1110 in the first through hole 1109 may be level with the depth of the first through hole 1109. In this way, the conductive material may be disposed on the top end surface of the support component 110, and the conductive material communicates with the first conductive adhesive 1110, to implement the electrical connection between the first conductive adhesive 1110 and the top end surface of the support component 110.

In an actual design, material usage of the first conductive adhesive 1110 disposed in the first through hole 1109 may also be effectively reduced by using the following example. By way of an example but not a limitation, when the support component 110 is made of a light-shielding insulation material or a light-transmitting material, the conductive material may alternatively be disposed on the inner side wall of the first through hole 1109, so that at least one conductive path is established between the first conductive adhesive 1110 and the top end surface of the support component 110. In this way, the disposition height of the first conductive adhesive 1110 in the first through hole 1109 may be set randomly.

It should be noted that in the foregoing structure in which the support component 110 is electrically connected to the first electrode 141 by injecting the first conductive adhesive 1110 into the first through hole 1109, the following two manners may be used to implement the electrical connection between the top end surface of the support component 110 and the first electrode 141. In one manner, the first through hole 1109 is filled with the first conductive adhesive 1110, and then a conductive path to the first conductive adhesive 1110 may be established based on the support component 110 in a manner, for example, in which the conductive material is disposed on the top end surface of the support component 110. In this way, during actual application, when the skin is in contact with the top end surface of the support component 110, the first electrode 141 may be electrically connected to the skin through the conductive path between the first conductive adhesive 1110 and the top end surface of the support component 110. In the other manner, the conductive material is directly disposed on the surface, of the support component 110, that includes the inner side wall of the first through hole 1109, so that the electrical connection path between the first conductive adhesive 1110 and the top end surface of the support component 110 can be established by disposing the first conductive adhesive 1110 of any height in the first through hole 1109. In this way, an electrical connection path can be formed between the first electrode 141, the first conductive adhesive 1110, and the top end surface of the support component 110.

It can be understood that in the foregoing several possible implementations, for the specific material and the structure disposition of the support component 110, refer to related descriptions such as the material and the structure of the support component 110 in the foregoing embodiment in which the support component 110 is directly electrically connected to the first electrode 141. Details are not described herein again.

Optionally, in the foregoing optional implementation, a shape of the first through hole 1109 provided on the support component 110 may be the same as a shape of the first electrode 141. Assuming that the shape of the first electrode 141 is a cuboid, the shape of the first through hole 1109 may also be a cuboid. In an actual design, the shape of the first through hole 1109 may also be different from the shape of the first electrode 141. For example, as shown in FIG. 10, the first through hole 1109 may include a large hole segment and a small hole segment, the large hole segment communicates with the small hole segment, one end that is of the large hole segment and that is away from the small hole segment wraps the first electrode 141, and the first conductive adhesive 1110 used to electrically connect the support component 110 to the first electrode 141 may be injected in both the large hole segment and the small hole segment.

In addition to the foregoing several possible implementations, the surface of the support component 110 may alternatively be directly covered with a conductive coating or conductive adhesive. When the FPC 140 is connected to the bottom end surface of the support component 110, an electrical connection path is formed between the top end surface of the support component 110 and the first electrode 141 by using the conductive coating or the conductive adhesive covering on the surface of the support component 110. In this way, a material of the support component 110 during actual application does not need to be considered; and in an actual manufacturing process, a specific disposition manner, a material, and the like of the conductive coating or the conductive adhesive on the top end surface of the support component 110 and the first electrode 141 that are electrically connected may be flexibly changed.

It should be understood that the structure of the support component 110 may be integrally formed into an integrated structure. In another possible implementation, the support component 110 may alternatively be formed by splicing a plurality of mechanical parts.

FIG. 12 is a diagram of a cross-sectional structure of a support component 110 formed by splicing two mechanical parts according to an embodiment of this application. Refer to FIG. 12. The support component 110 includes a base 112 and a first mechanical part 111. A top end surface of the base 112 is fastened to a bottom end surface of the first mechanical part 111. A first groove 1101 and a second groove 1102 are provided on a bottom end surface of the base 112. A second light exit window 1111 corresponding to the first groove 1101 and a second light entry window 1112 corresponding to the second groove 1102 are provided on the first mechanical part 111. The second light exit window 1111 communicates with the first groove 1101, and the second light exit window 1111 is used for emission of a light ray emitted by the optical transmitter 130 disposed in the first groove 1101. The second light entry window 1112 communicates with the second groove 1102, and the second light entry window 1112 is configured to receive a reflected light ray by the optical receiver 120 disposed in the second groove 1102.

The bottom end surface of the base 112 in the support component 110 is fastened to the FPC 140, to form a first accommodating cavity 150 corresponding to the first groove 1101 and a second accommodating cavity 160 corresponding to the second groove 1102. The optical transmitter 130 is disposed in the first accommodating cavity 150, the optical receiver 120 is disposed in the second accommodating cavity 160, both the optical transmitter 130 and the optical receiver 120 are connected to the FPC 140, and a first electrode 141 is further disposed on the FPC 140, and the first electrode 141 is electrically connected to the first mechanical part 111 through the base 112.

To avoid a large splicing error of a plurality of mechanical parts of the support component 110, which reduces utilization of the light ray emitted by the optical transmitter 130 and utilization of the reflected light ray received by the optical receiver 120, in an optional design manner, a first hole segment corresponding to the first groove 1101 and a second hole segment corresponding to the second groove 1102 may be further separately disposed on the top end surface of the base 112, the first hole segment correspondingly communicates with the second light exit window 1111 on the first mechanical part 111, and the second hole segment correspondingly communicates with the second light entry window 1112 on the first mechanical part 111. In this way, not only alignment between the base 112 and the first mechanical part 111 is facilitated, but also selection of materials for manufacturing the support component 110 may be further enriched.

Optionally, the first mechanical part 111 may include a first mechanical part body, and the first mechanical part body is made of a conductive light-shielding material.

It should be understood that when the first mechanical part body is made of a conductive light-shielding material, the first mechanical part 111 may be directly electrically connected to the first electrode 141 through the base 112.

In a possible implementation, the first mechanical part 111 includes a first mechanical part body and a first conductive coating 1106 disposed on a surface of the first mechanical part body, and the first mechanical part body is made of a light-shielding insulation material.

It should be understood that when the first mechanical part body is made of a light-shielding insulation material, the first mechanical part 111 may be electrically connected to the first electrode 141 by using the first conductive coating 1106 disposed on the surface of the first mechanical part body and the base 112.

In another possible implementation, the first mechanical part 111 includes a first mechanical part body and a conductive light-shielding layer disposed on a surface of the first mechanical part body, and the first mechanical part body is made of a light-transmitting material.

It should be understood that when the first mechanical part body is made of a light-transmitting material, the first mechanical part 111 may be electrically connected to the first electrode 141 through the conductive light-shielding layer disposed on the surface of the first mechanical part body and the base 112.

In another possible implementation, the first mechanical part 111 includes a first mechanical part body, a light-shielding insulation layer 1107 disposed on a surface of the first mechanical part body, and a second conductive coating 1108 disposed on a surface of the light-shielding insulation layer 1107, and the first mechanical part body is made of a light-transmitting material.

It should be understood that when the first mechanical part body is made of a light-transmitting material, the first mechanical part 111 may be electrically connected to the first electrode 141 by using the second conductive coating 1108 on the surface of the light-shielding insulation layer 1107 and the base 112.

It should be noted that, as shown in FIG. 12, in a manner in which the first electrode 141 is directly electrically connected to the first mechanical part 111 through the base 112, for a specific structure of the first mechanical part 111, refer to the foregoing several possible implementations. In addition, based on the foregoing several possible specific structures of the first mechanical part 111, in an example in which the first mechanical part 111 is electrically connected to the first electrode 141 through the base 112, a specific structure of the base 112 may include the following several possible implementations.

Optionally, the base 112 may include a base body, and the base body is made of a conductive light-shielding material.

In a possible implementation, the base 112 includes a base body and the first conductive coating 1106 disposed on a surface of the base body, and the base body is made of a light-shielding insulation material.

In another possible implementation, the base 112 includes a base body and a conductive light-shielding layer disposed on a surface of the base body, and the base body is made of a light-transmitting material.

In another possible implementation, the base 112 includes a base body, the light-shielding insulation layer 1107 disposed on a surface of the base body, and a second conductive coating 1108 disposed on the surface of the light-shielding insulation layer 1107, and the base body is made of a light-transmitting material.

It should be understood that, based on an example in which the support component 110 is formed by splicing the base 112 and the first mechanical part 111, as shown in FIG. 12, the second light exit window 1111 may be a second through hole provided on the first mechanical part 111, and/or the second light entry window 1112 may be a third through hole provided on the first mechanical part 111. In other words, the second light exit window 1111 and/or the second light entry window 1112 may be through holes. In this way, utilization of the light ray emitted by the optical transmitter 130 and/or the light ray received by the optical receiver 120 can be improved.

Refer to FIG. 12. Optionally, the second light exit window 1111 may be the second through hole provided on the first mechanical part 111, and a first transparent part is disposed in the second through hole; and/or the second light entry window 1112 may be the third through hole provided on the first mechanical part 111, and a second transparent part is disposed in the third through hole.

It should be noted that when the first mechanical part body is made of a light-transmitting material, a material of the second light exit window 1111 provided on the first mechanical part 111 may be the same as a material of the first mechanical part body, and/or a material of the second light entry window 1112 provided on the first mechanical part 111 may be the same as the material of the first mechanical part body. In other words, the second through hole and/or the third through hole do/does not need to be provided on the first mechanical part 111, and the second light exit window 1111 and the second light entry window 1112 are regions that are on the first mechanical part body and that are not coated with materials that are both light-shielding and conductive, for example, the first conductive coating 1106 that is light-shielding, the conductive light-shielding layer, the light-shielding insulation layer 1107, and the second conductive coating 1108.

To effectively ensure accuracy of health measurement performed through the optical transmitter 130 and the optical receiver 120, in a possible implementation, transmittance of the second light exit window 1111 and/or transmittance of the second light entry window 1112 may be greater than 90%. It should be understood that the material of the first light exit window 1103, the material of the first light entry window 1104, the material of the second light exit window 1111, and the material of the second light entry window 1112 may be the same, or may be partially the same, or may be completely different. This is not limited in this application.

In the foregoing embodiment, light transmitting of the first conductive coating 1106, light transmitting of the second conductive coating 1108, a specific disposition manner of the first conductive coating 1106 or the second conductive coating 1108 in the second light exit window 1111 and/or the second light entry window 1112, specific types of the first transparent part and the second transparent part disposed in the second light exit window 1111 and/or the second light entry window 1112, a volume size of the first groove 1101 and a volume size of the second groove 1102 provided on the bottom end surface of the base 112, whether a specific type of the first transparent part disposed in the second through hole is the same as a specific type of the second transparent part disposed in the third through hole, a specific type of a transparent part disposed in the first groove 1101, a disposition thickness and a specific type of a transparent part disposed in the second groove 1102, a disposition thickness and specific types of transparent parts disposed in the second through hole, the third through hole, the first groove 1101, and the second groove 1102, whether disposition thicknesses are the same, transmittance of the second light exit window 1111, transmittance of the second light entry window 1112, and/or the like, refer to related descriptions in the foregoing embodiment in which the support component 110 is made of a conductive light-shielding material. Details are not described herein again.

It should be noted that, as shown in FIG. 12, the second light exit window 1111 and/or the second light entry window 1112 that are/is provided on the first mechanical part 111 is only a possible implementation. In an actual structure design, a specific structure design of the second light exit window 1111 and/or the second light entry window 1112 may also be provided on both the first mechanical part 111 and the base 112. In other words, a distance between the top end surface and the bottom end surface of the second light exit window 1111 (namely, a height or a thickness of the second light exit window 1111) is greater than a thickness of the first mechanical part 111, and/or a distance between the top end surface and the bottom end surface of the second light entry window 1112 is also greater than the thickness of the first mechanical part 111. This is not limited in this application.

For the structure of the support component 110 shown in FIG. 12 that is formed by splicing the base 112 and the first mechanical part 111, a specific material and structure disposition of the first mechanical part 111 in the support component 110, and/or a specific material and structure disposition of the base 112, refer to related descriptions of various different structures of the support component 110 in the foregoing embodiment in which the support component 110 is integrally formed. Details are not described herein again. In an embodiment in which the support component 110 is formed by splicing the base 112 and the first mechanical part 111, specific materials and structure disposition of the first mechanical part 111 and the base 112 meet that at least one electrical connection path may be directly established between the top end surface of the first mechanical part 111 and the first electrode 141, or at least one electrical connection path may be established between the top end surface of the first mechanical part 111 and the first electrode 141 through the base 112.

In a possible implementation, as shown in FIG. 12, the bottom end surface of the base 112 in the support component 110 is directly electrically connected to the first electrode 141. When the skin is in contact with the top end surface of the first mechanical part 111, a conductive path is formed between the first mechanical part 111, the base 112, and the first electrode 141, and the first electrode 141 is directly electrically connected to the skin through the base 112 and the first mechanical part 111.

In another possible implementation, as shown in FIG. 13, a conductive portion 1105 is extended and disposed on the base 112, and the top end surface of the first mechanical part 111 is electrically connected to the first electrode 141 through the base 112 and the conductive portion 1105.

It should be understood that, for a specific material of the conductive portion 1105, refer to a disposition status of the material of the conductive portion 1105 in the embodiment in which the support component 110 is of an integrated structure for understanding. For a specific structure of the base 112 and a specific structure of the first mechanical part 111, refer to the specific structure of the base 112 and the specific structure of the first mechanical part 111 in the embodiment in which the support component 110 is formed by splicing the base 112 and the first mechanical part 111 shown in FIG. 12. Details are not described herein again.

Specifically, as shown in FIG. 13, the conductive portion 1105 is extended and disposed on the bottom end surface of the base 112, the conductive portion 1105 is located between the first groove 1101 and the second groove 1102, the first electrode 141 corresponding to the conductive portion 1105 is disposed on the FPC 140, and the conductive portion 1105 has conductivity. In this way, the first electrode 141 may be electrically connected to the first mechanical part 111 through the conductive portion 1105 and the base 112. Based on this implementation, when the skin is in contact with the top end surface of the first mechanical part 111, the first electrode 141 may be connected to the skin through the conductive path formed between the first mechanical part 111, the base 112, the conductive portion 1105, and the first electrode 141.

In still another possible implementation, as shown in FIG. 14, for a case in which the support component 110 includes two mechanical parts: the base 112 and the first mechanical part 111, a fourth through hole used to accommodate the elastic component 170 is further provided on the base 112, and the first mechanical part 111 is electrically connected to the first electrode 141 through the elastic component 170 disposed in the fourth through hole.

In this implementation, for a specific disposition manner, a disposition location, and a specific type of the elastic component 170, refer to the disposition status of the elastic component 170 in the embodiment in which the support component 110 is of an integrated structure for understanding. Details are not described herein again in this application.

It should be noted that, based on the implementation shown in FIG. 14, in addition to the specific structure of the base 112 in the embodiment shown in FIG. 12 or FIG. 13, the specific structure of the base 112 may also be disposed randomly, because disposition of the specific structure of the base 112 does not affect the electrical connection between the first mechanical part 111 and the first electrode 141.

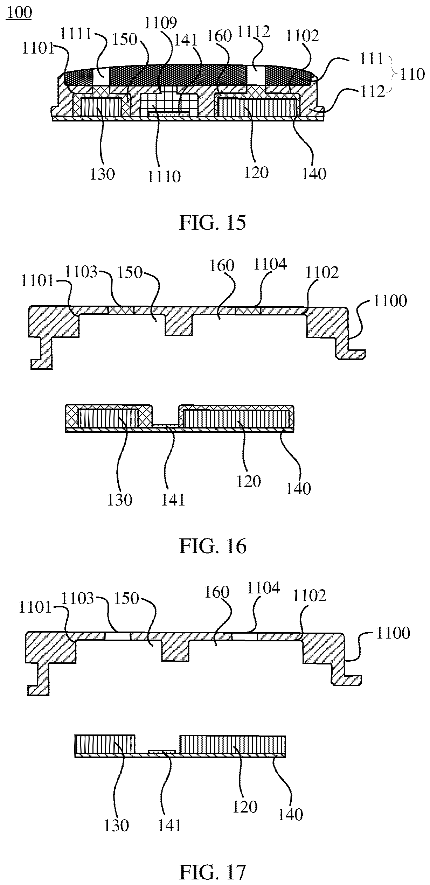

In another possible implementation, as shown in FIG. 15, based on disposition of the structure in which the support component 110 includes the base 112 and the first mechanical part 111, the first through hole 1109 penetrates through the top end surface and the bottom end surface of the base 112, the first conductive adhesive 1110 is injected into the first through hole 1109, and the top end surface of the first mechanical part 111 is electrically connected to the first electrode 141 through the first mechanical part 111 and the first conductive adhesive 1110.

It is not difficult to understand that, based on the possible example in FIG. 15, the specific structure of the base 112 may also be disposed randomly. For example, in a case in which the specific structure of the base 112 is not limited, the first through hole 1109 may be filled with the first conductive adhesive 1110, so that the first conductive adhesive 1110 is in contact with the bottom end surface of the first mechanical part 111. When the first mechanical part 111 has conductivity, the first electrode 141 may be electrically connected to the first mechanical part 111 through the first conductive adhesive 1110.

In this example, a groove or a through hole corresponding to the first through hole 1109 may be further provided on the first mechanical part 111, and a first conductive adhesive 1110 is disposed in the groove or the through hole, so that the top end surface of the first mechanical part 111 is electrically connected to the first electrode 141 through the first conductive adhesive 1110 disposed in the groove or the through hole and the first conductive adhesive 1110 disposed in the first through hole 1109.

It should be understood that, in this example, for specific disposition of the first through hole 1109 and the first conductive adhesive 1110, refer to the disposition status of the first conductive adhesive 1110 disposed in the first through hole 1109 in the embodiment in which the support component 110 is of an integrated structure for understanding. Details are not described herein again in this application.

Certainly, in an actual design, the support component 110 may alternatively be formed by splicing a plurality of other mechanical parts. In this embodiment of this application, a specific quantity or quantity of layers of the mechanical parts in the support component 110, a specific structure of the support component 110, and specific disposition structures of the second light exit window 1111, the second light entry window 1112, the first groove 1101, and the second groove 1102 on the mechanical parts are not limited. It is not difficult to understand that, in comparison with disposition of the integrated structure of the support component 110, in the disposition manner in which the support component 110 uses the plurality of mechanical parts for splicing, the material of the base 112 is relatively flexible, and the structure of the first mechanical part 111 is relatively simple and easy to implement.

An example in which the support component 110 is of an integrated structure, a process of disposing an integrated structure of the detection apparatus 100 may include but is not limited to the following: In a first manner, as shown in FIG. 16, the first light exit window 1103 and the first light entry window 1104 on the support component 110 are windows with transparent parts; the optical transmitter 130, the optical receiver 120, and the first electrode 141 are disposed on the FPC 140 to form a first integrated structure, and the support component 110 and the first integrated structure are fastened to form the detection apparatus 100 provided in this embodiment of this application. This manner separately facilitates modification and adjustment of the support component 110 and the first integrated structure.

In a second manner, as shown in FIG. 17, the first light exit window 1103 and the first light entry window 1104 that are provided on the support component 110 respectively correspond to two through holes; the optical transmitter 130, the optical receiver 120, and the first electrode 141 are disposed on the FPC 140 to form a first integrated structure; and after the support component 110 is paired with the first integrated structure, a transparent part such as a transparent adhesive is injected into the first groove 1101, the second groove 1102, and the two through hole through the two through hole, so that the support component 110 is fastened to the first overall structure. In this disposition manner, the fixed connection between the support component 110 and the first integrated structure can be further strengthened in a disposition manner of injecting the transparent part, and stability of the detection apparatus 100 and strength of the mechanical structure can be improved.

Optionally, the detection apparatus 100 may be injection-molded into an integrated structure in a lowtemperature and low-pressure injection molding manner.

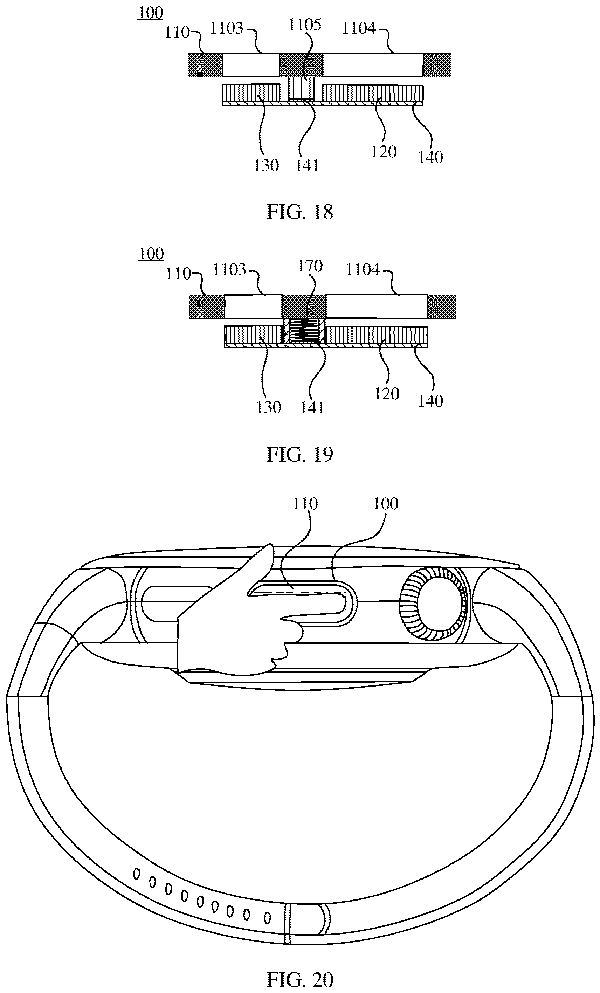

In a possible implementation, FIG. 18 is another diagram of a cross-sectional structure of a part of a detection apparatus 100 after the detection apparatus 100 is disposed in an electronic device according to an embodiment of this application. Refer to FIG. 18. The detection apparatus 100 is embedded in the electronic device. The detection apparatus 100 is configured to detect and obtain health detection data such as blood pressure and a heart rate when skin is in contact with a top end surface of the detection apparatus 100. The detection apparatus 100 is embedded on an outer surface of a side wall of a housing. The electronic device includes the housing, a support component 110, an optical receiver 120, an optical transmitter 130, and an FPC 140. The FPC 140 is disposed in the housing, and both the optical receiver 120 and the optical transmitter 130 are electrically connected to the FPC 140. The support component 110 is embedded on the outer surface of the side wall of the housing, a first light exit window 1103 corresponding to the optical transmitter 130 and a first light entry window 1104 corresponding to the optical receiver 120 are provided on the support component 110, the first light exit window 1103 is used for emission of a light ray emitted by the optical transmitter 130, and the first light entry window 1104 is configured to receive a reflected light ray by the optical receiver 120. A first electrode 141 is further disposed on the FPC 140, the FPC 140 is configured to provide an electrical signal for the optical transmitter 130, the optical receiver 120, and the first electrode 141, and the first electrode 141 is electrically connected to the support component 110. A second electrode is embedded on a bottom wall of the housing, and the second electrode and the first electrode 141 are configured to form a path to collect the electrical signal when skin is separately in contact with the top end surface of the support component 110 and the bottom wall of the housing.

In this embodiment of this application, a specific structure of the support component 110 may be the structure of the support component 110 in any one of the foregoing embodiments. Certainly, the specific structure of the support component 110 may also be a support plate embedded in the housing. A projection shape of the support plate on the FPC 140 may be a rectangle, a square, a runway shape, an ellipse, any polygon, or the like. A first light exit window 1103 and a first light entry window 1104 are provided on the support plate, and there is at least one electrical connection path between a top end surface of the support plate and the first electrode 141.

It should be understood that, in this implementation, the support component 110 in the detection apparatus 100 is directly embedded in the housing of the electronic device, the FPC 140 is directly disposed in the electronic device, and the electronic device has a health detection function through mutual cooperation between the support component 110 and the FPC 140. In comparison with the foregoing independent and complete detection apparatus 100, the detection apparatus 100 in the foregoing implementation has strong dependency on a structure of the electronic device, and assembly of the detection apparatus 100 needs to be implemented by using the structure of the electronic device.

To avoid impact between the light ray emitted by the optical transmitter 130 and the light ray received by the optical receiver 120, and improve accuracy of health detection data of the detection apparatus 100, in a possible implementation, distances between the optical transmitter 130 and the optical receiver 120 that are disposed on the FPC 140 and the support component 110 needs to be as close as possible. Specifically, a distance between the optical transmitter 130 and the first light exit window 1103 is as close as possible, so that the light ray emitted by the optical transmitter 130 can be fully used, and accuracy of an optical signal received by the skin can be improved; and/or a distance between the optical receiver 120 and the first light entry window 1104 is as close as possible, so that the optical receiver 120 receives a more accurate emitted light ray, and accuracy of obtaining health detection data is improved.

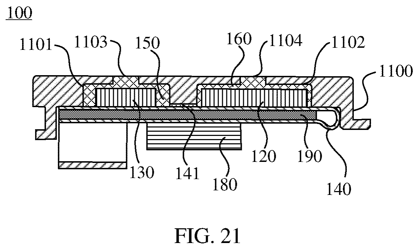

Based on the foregoing structure disposition, it is not difficult to see that the support component 110 and the FPC 140 are two modules independent of each other. To effectively implement the electrical connection between the support component 110 and the first electrode 141 disposed on the FPC 140 based on this structure, in a possible implementation, as shown in FIG. 21, a conductive portion 1105 is extended and disposed on the support component 110, and the top end surface of the support component 110 or the support component 110 is electrically connected to the first electrode 141 through the conductive portion 1105.

It should be understood that the conductive portion 1105 has conductivity. The conductive portion 1105 includes a conductive portion body. The conductive portion body or the conductive portion 1105 may be made of a conductive (light-shielding or light transmitting) material, and the conductive portion 1105 is directly electrically connected to the first electrode 141 through abutment or a rigid connection between a bottom end surface of the conductive portion 1105 and a top end surface of the first electrode 141. The conductive portion body may also be a (light-shielding or light transmitting) insulation material, and the conductive portion 1105 has conductivity by disposing a conductive material on a surface of the conductive portion body.

It should be noted that, as shown in FIG. 18, if the conductive portion 1105 is disposed between the optical transmitter 130 and the optical receiver 120, and the conductive portion body is made of a light-transmitting material, a light-shielding material may be coated on the surface of the conductive portion body, and the conductive material is disposed on a surface of the light-shielding material. In this way, the conductive portion 1105 not only has conductivity but also is light-shielding, can effectively separate the light ray emitted by the optical transmitter 130 from the light ray received by the optical receiver 120, and can also implement the electrical connection between the conductive portion 1105 and the first electrode 141.

In the foregoing embodiment, for a specific case in which the support component 110 includes a material of the support component body, the conductive portion 1105 includes a material of the conductive portion body, whether materials of the conductive portion body and the support component body are the same, and whether the first electrode 141 is electrically connected to the top end surface of the support component 110 through the conductive portion 1105, refer to related descriptions of the electrical connection between the first electrode 141 and the support component 110 through the conductive portion 1105 in the foregoing embodiment in which the support component 110 is formed into an integrated structure. Details are not described herein again.

In another possible implementation, as shown in FIG. 19, the electronic device further includes an elastic component 170. One end of the elastic component 170 is electrically connected to a bottom end surface of the support component 110, and the other end of the elastic component 170 is electrically connected to the first electrode 141. When skin is in contact with the top end surface of the support component 110, the top end surface of the support component 110 is electrically connected to the first electrode 141 through the support component 110 and the elastic component 170, and the first electrode 141 may be electrically connected to the skin through the foregoing electrical connection path.

It should be noted that, as shown in FIG. 19, a light-blocking component may be further disposed between the two sides of the elastic component 170. The light-blocking component is configured to separate an optical signal transmitted by the optical transmitter 130 from an optical signal received by the optical receiver 120. Such disposition can avoid a case in which detection data is inaccurate because the optical signal transmitted by the optical transmitter 130 and the optical signal received by the optical receiver 120 affect each other.

In this possible embodiment, for a specific disposition status of the material of the support component body included in the support component 110 and a disposition location of the elastic component 170, refer to related descriptions of the specific disposition material of the support component body and the electrical connection between the first electrode 141 and the top end surface of the support component 110 through the elastic component 170 in the foregoing embodiment in which the support component 110 is formed into an integrated structure. Details are not described herein again.

In an actual design, based on different actual application scenarios of the detection apparatus 100 or different structure designs of the electronic device, at least one optical transmitter 130 and/or at least one optical receiver 120 may be disposed in the detection apparatus 100. Similarly, at least one first electrode 141 may also be disposed in the detection apparatus 100. Quantities of optical transmitters 130, optical receivers 120, and first electrodes 141 disposed in the detection apparatus 100 are not limited in this application.

FIG. 20 is a diagram of an actual application scenario in which a detection apparatus 100 is disposed in a smartwatch according to an embodiment of this application. Refer to FIG. 20. The detection apparatus 100 is installed on an outer side wall of the smartwatch, and a second electrode is further embedded on a bottom wall of a housing of the smartwatch. After a user wears the smartwatch on a wrist, the wrist may be electrically connected to the second electrode. When a finger of the user is placed on or in contact with a top end surface of a support component 110 in the detection apparatus 100, the optical transmitter 130 disposed in the detection apparatus 100 emits a PPG signal (namely, an optical signal), and the PPG signal reflected by the finger of the user is received by the optical receiver 120. In addition, the finger placed on the top end surface of the support component 110 is electrically connected to the first electrode 141 through the support component 110, and an electrical connection between the first electrode 141 and the second electrode is implemented through the finger and the wrist. In this way, electrical signals of the finger and the wrist of the user can be collected by the first electrode 141 and the second electrode that are electrically connected. The electrical signals collected by the first electrode 141 and the second electrode and the optical signal received by the optical receiver 120 are transmitted to a processor in the smartwatch through an FPC 140. After being processed by the processor, health detection data such as blood pressure, a heart rate, and blood oxygen saturation may be generated, and the health detection data is displayed on a display of the smartwatch, and/or the health detection data is stored in a memory of the smartwatch or a cloud server corresponding to the smartwatch.

To further enrich function disposition in the electronic device through the detection apparatus 100, in this embodiment of this application, as shown in FIG. 21, the detection apparatus 100 further includes a key Dome sheet 180 and a support structure 190, the FPC 140 is disposed around the support structure 190, the key Dome sheet 180 is disposed on a first surface of the FPC 140, the optical receiver 120 is disposed on a second surface of the FPC 140, and the first surface and the second surface are disposed opposite to each other.

It should be understood that, if the key Dome sheet 180 and the support structure 190 are not disposed in the detection apparatus 100, after the detection apparatus 100 is embedded in a terminal device, the detection apparatus 100 is a touch key, that is, health detection may be performed after the skin is in contact with the top end surface of the support component 110 in the detection apparatus 100. If the key Dome sheet 180 and the support structure 190 are disposed in the detection apparatus 100, after the detection apparatus 100 is disposed in the terminal device, the detection apparatus 100 on the terminal device is a press button.

After the detection apparatus 100 on which the key Dome sheet 180 and the support structure 190 are disposed is assembled on the smartwatch shown in FIG. 20, a press function of the detection apparatus 100 may be used to implement disposition of different functions in the smartwatch. For example, the user may press the detection apparatus 100 disposed on the smartwatch, so that a display interface of the smartwatch goes to function interfaces such as a health management interface, a movement mode interface, or a voice broadcast health detection result interface. In this design manner, an application scope of the detection apparatus 100 can be further enriched, and practicability of the detection apparatus 100 is improved.

Certainly, during actual application, to implement an anti-accidental touch function between the skin and the detection apparatus 100, after the user places the finger on the detection apparatus 100 and presses the detection apparatus 100, the detection apparatus 100 may be triggered to collect the corresponding optical signal and the electrical signal to detect various physiological parameters of the user. This helps save electric energy of the smartwatch and prolong a service life of the smartwatch.

An example in which the support component 110 is of an integrated structure is used. An embodiment of this application provides sizes of components in the detection apparatus 100. The sizes of the following components are merely design parameters that can be specifically implemented and that are provided in this embodiment of this application. In an actual processing and production process, the sizes of the components may be appropriately increased to improve product yield rates of the detection apparatus 100 and the electronic device, or the sizes of the components may be appropriately decreased to enable the detection apparatus 100 to be applicable to another miniaturized and lightweight electronic device.

Optionally, a thickness of the support structure 190 is approximately 0.2 millimeters.

Optionally, a side length of a long side edge of the support component 110 is approximately 11.55 millimeters, a side length of a short side edge of the support component 110 is approximately 2.8 millimeters, and a thickness of the support component 110 is approximately 2.2 millimeters.

Optionally, a thickness of the FPC 140 is approximately 0.12 millimeters.

Optionally, a thickness of the first light exit window 1103 and/or a thickness of the first light entry window 1104 are/is approximately 0.2 millimeters.

Optionally, a side length of a long side edge of the optical transmitter 130 is approximately 1.85 millimeters, and a thickness of the optical transmitter 130 is approximately 0.6 millimeters; and a side length of a long side edge of the optical receiver 120 is approximately 2.6 millimeters, and a thickness of the optical receiver 120 is approximately 0.6 millimeters.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

**Claims**

1. A detection apparatus, wherein the detection apparatus (100) comprises a support component (110), an optical receiver (120), an optical transmitter (130), and a flexible printed circuit FPC (140);

   a first groove (1101) and a second groove (1102) are provided on a bottom end surface of the support component (110), the bottom end surface of the support component (110) is connected to the FPC (140), to form a first accommodating cavity (150) corresponding to the first groove (1101) and a second accommodating cavity (160) corresponding to the second groove (1102), a first light exit window (1103) and a first light entry window (1104) are provided on a top end surface of the support component (110), the first groove (1101) communicates with the first light exit window (1103), and the second groove (1102) communicates with the first light entry window (1104); the optical transmitter (130) and the optical receiver (120) are both electrically connected to the FPC (140), the optical transmitter (130) is disposed in the first accommodating cavity (150), and the optical receiver (120) is disposed in the second accommodating cavity (160); and a first electrode (141) is disposed on the FPC (140), and the first electrode (141) is electrically connected to the support component (110).

2. The detection apparatus according to claim 1, wherein a conductive portion (1105) is disposed on the support component (110), and the support component (110) is electrically connected to the first electrode (141) through the conductive portion (1105).

3. The detection apparatus according to claim 1, wherein the detection apparatus (100) further comprises an elastic component (170), a third accommodating cavity configured to accommodate the elastic component (170) is provided on the support component (110), and the support component (110) is electrically connected to the first electrode (141) through the elastic component (170) disposed in the third accommodating cavity.

4. The detection apparatus according to claim 1, wherein a first through hole (1109) corresponding to the first electrode (141) is provided on the support component (110), a first conductive adhesive (1110) is disposed in the first through hole (1109), and the support component (110) is electrically connected to the first electrode (141) through the first conductive adhesive (1110).

5. The detection apparatus according to any one of claims 1 to 4, wherein the support component (110) comprises a support component body (1100), and the support component body (1100) is made of a conductive light-shielding material.

6. The detection apparatus according to any one of claims 1 to 4, wherein the support component (110) comprises a support component body (1100) and a first conductive coating (1106) disposed on a surface of the support component body (1100), and the support component body (1100) is made of a light-shielding insulation material.

7. The detection apparatus according to claim 6, wherein the first conductive coating (1106) is light-transmitting.

8. The detection apparatus according to claim 7, wherein an orthographic projection of the first conductive coating (1106) on the support component body (1100) covers the light exit window (1103) and/or the light entry window (1104).

9. The detection apparatus according to any one of claims 1 to 4, wherein the support component (110) comprises a support component body (1100) and a conductive light-shielding layer disposed on a surface of the support component body (1100), and the support component body (1100) is made of a light-transmitting material.

10. The detection apparatus according to any one of claims 1 to 4, wherein the support component (110) comprises a support component body (1100), a light-shielding insulation layer (1107) disposed on a surface of the support component body (1100), and a second conductive coating (1108) disposed on a surface of the light-shielding insulation layer (1107), and the support component body (1100) is made of a light-transmitting material.

11. The detection apparatus according to claim 10, wherein the second conductive coating (1108) is light-transmitting.

12. The detection apparatus according to claim 11, wherein an orthographic projection of the second conductive coating (1108) on the support component body (1100) covers the first light exit window (1103) and/or the first light entry window (1104).

13. The detection apparatus according to any one of claims 1 to 12, wherein the first light exit window (1103) is a second through hole provided on the support component (110), and/or the first light entry window (1104) is a third through hole provided on the support component (110).

14. The detection apparatus according to claim 13, wherein a first transparent part is disposed in the second through hole, and/or a second transparent part is disposed in the third through hole.

15. The detection apparatus according to any one of claims 1 to 14, wherein the support component (110) is of an integrated structure.

16. The detection apparatus according to any one of claims 1 to 15, wherein transmittance of the first light exit window (1103) and/or transmittance of the first light entry window (1104) are/is greater than 90%.

17. A detection apparatus, wherein the detection apparatus (100) comprises a support component (110), an optical receiver (120), an optical transmitter (130), and an FPC (140);

the support component (110) comprises a first mechanical part (111) and a base (112), the first mechanical part (111) is fastened to the base (112), a first groove (1101) and a second groove (1102) are provided on a bottom end surface of the base (112), a second light exit window (1111) and a second light entry window (1112) are provided on the first mechanical part (111), the first groove (1101) communicates with the second light exit window (1111), and the second groove (1102) communicates with the second light entry window (1112);
the base (112) is fastened to the FPC (140), and forms a first accommodating cavity (150) and a second accommodating cavity (160) with the FPC (140), the first accommodating cavity (150) corresponds to the first groove (1101), and the second accommodating cavity (160) corresponds to the second groove (1102);
the optical transmitter (130) and the optical receiver (120) are both electrically connected to the FPC (140), the optical transmitter (130) is disposed in the first accommodating cavity (150), and the optical receiver (120) is disposed in the second accommodating cavity (160); and
a first electrode (141) is disposed on the FPC (140), and the first electrode (141) is electrically connected to the first mechanical part (111) through the base (112).

18. The detection apparatus according to claim 17, wherein a conductive portion (1105) is disposed on the base (112), and the first mechanical part (111) is electrically connected to the first electrode (141) through the base (112) and the conductive portion (1105).

19. The detection apparatus according to claim 17, wherein the detection apparatus (100) further comprises an elastic component (170), a third accommodating cavity configured to accommodate the elastic component (170) is provided on the base (112), and the first mechanical part (111) is electrically connected to the first electrode (141) through the elastic component (170) disposed in the third accommodating cavity.

20. The detection apparatus according to claim 17, wherein a first through hole (1109) corresponding to the first electrode (141) is provided on the base (112), a first conductive adhesive (1110) is disposed in the first through hole (1109), and the first mechanical part (111) is electrically connected to the first electrode (141) through the first conductive adhesive (1110).

21. The detection apparatus according to any one of claims 17 to 20, wherein the first mechanical part (111) comprises a first mechanical part body, and the first mechanical part body is made of a conductive light-shielding material.

22. The detection apparatus according to any one of claims 17 to 20, wherein the first mechanical part (111) comprises a first mechanical part body and a first conductive coating (1106) disposed on a surface of the first mechanical part body, and the first mechanical part body is made of a light-shielding insulation material.

23. The detection apparatus according to claim 22, wherein the first conductive coating (1106) is light-transmitting.

24. The detection apparatus according to claim 23, wherein an orthographic projection of the first conductive coating (1106) on the first mechanical part body covers the second light exit window (1111) and/or the second light entry window (1112).

25. The detection apparatus according to any one of claims 17 to 20, wherein the first mechanical part (111) comprises a first mechanical part body and a conductive light-shielding layer disposed on a surface of the first mechanical part body, and the first mechanical part body is made of a light-transmitting material.

26. The detection apparatus according to any one of claims 17 to 20, wherein the first mechanical part (111) comprises a first mechanical part body, a light-shielding insulation layer (1107) disposed on a surface of the first mechanical part body, and a second conductive coating (1108) disposed on a surface of the light-shielding insulation layer (1107), and the first mechanical part body is made of a light-transmitting material.

27. The detection apparatus according to claim 26, wherein the second conductive coating (1108) is light-transmitting.

28. The detection apparatus according to claim 27, wherein an orthographic projection of the second conductive coating (1108) on the first mechanical part body covers the second light exit window (1111) and/or the second light entry window (1112).

29. The detection apparatus according to any one of claims 17 to 28, wherein the base (112) comprises a base body, and the base body is made of a conductive light-shielding material.

30. The detection apparatus according to any one of claims 17 to 28, wherein the base (112) comprises a base body and the first conductive coating (1106) disposed on a surface of the base body, and the base body is made of a light-shielding insulation material.

31. The detection apparatus according to claim 30, wherein the first conductive coating (1106) is light-transmitting.

32. The detection apparatus according to claim 31, wherein an orthographic projection of the first conductive coating (1106) on the base body covers the second light exit window (1111) and/or the second light entry window (1112).

33. The detection apparatus according to any one of claims 17 to 28, wherein the base (112) comprises a base body and a conductive light-shielding layer disposed on a surface of the base body, and the base body is made of a light-transmitting material.

34. The detection apparatus according to any one of claims 17 to 28, wherein the base (112) comprises a base body, the light-shielding insulation layer (1107) disposed on a surface of the base body, and the second conductive coating (1108) disposed on the surface of the light-shielding insulation layer (1107), and the base body is made of a light-transmitting material.

35. The detection apparatus according to claim 34, wherein the second conductive coating (1108) is light-transmitting.

36. The detection apparatus according to claim 35, wherein an orthographic projection of the second conductive coating (1108) on the base body covers the second light exit window (1111) and/or the second light entry window (1112).

37. The detection apparatus according to any one of claims 17 to 36, wherein the second light exit window (1111) is a second through hole provided on the first mechanical part (111), and/or the second light entry window (1112) is a third through hole provided on the first mechanical part (111).

38. The detection apparatus according to claim 37, wherein a first transparent part is disposed in the second through hole, and/or a second transparent part is disposed in the third through hole.

39. The detection apparatus according to any one of claims 17 to 38, wherein transmittance of the second light exit window (1111) and/or transmittance of the second light entry window (1112) are/is greater than 90%.

40. The detection apparatus according to any one of claims 1 to 39, wherein the detection apparatus (100) further comprises a key Dome sheet (180) and a support structure (190), the FPC (140) is disposed around the support structure (190), the key Dome sheet (180) is disposed on a first surface of the FPC (140), the optical receiver (120) is disposed on a second surface of the FPC (140), and the first surface and the second surface are disposed opposite to each other.

41. The detection apparatus according to any one of claims 1 to 40, wherein the detection apparatus (100) is of an integrated structure.

42. An electronic device, wherein the electronic device comprises a housing and the detection apparatus (100) according to any one of claims 1 to 41, and the detection apparatus (100) is embedded on an outer surface of a side wall of the housing.

43. The electronic device according to claim 42, wherein a second electrode is embedded on a bottom wall of the housing, and the second electrode and the first electrode (141) are configured to form a path to collect an electrical signal when skin is separately in contact with the top end surface of the support component (110) and the bottom wall of the housing.

44. An electronic device, wherein the electronic device comprises a housing, a support component (110), an optical receiver (120), an optical transmitter (130), and an FPC (140), wherein

the FPC (140) is disposed in the housing, and the optical receiver (120) and the optical transmitter (130) are both electrically connected to the FPC (140);
the support component (110) is embedded on an outer surface of a side wall of the housing, and a light exit window (1103) corresponding to the optical transmitter (130) and a light entry window (1104) corresponding to the optical receiver (120) are provided on the support component (110);
a first electrode (141) is further disposed on the FPC (140), and the first electrode (141) is electrically connected to the support component (110); and
a second electrode is embedded on a bottom wall of the housing, and the second electrode and the first electrode (141) are configured to form a path to collect an electrical signal when skin is separately in contact with the top end surface of the support component (110) and the bottom wall of the housing.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

## INTERNATIONAL SEARCH REPORT

| International application No. |
| --- |
| **PCT/CN2024/078320** |

**A.    CLASSIFICATION OF SUBJECT MATTER**

A61B5/0205(2006.01)i

According to International Patent Classification (IPC) or to both national classification and IPC

**B.    FIELDS SEARCHED**

Minimum documentation searched (classification system followed by classification symbols)

IPC:A61B 5

Documentation searched other than minimum documentation to the extent that such documents are included in the fields searched

Electronic data base consulted during the international search (name of data base and, where practicable, search terms used)

CNTXT, ENTXT, VEN: 穿戴, 弹, 电极, 电路板, 光电, 脉搏, 手表, 手环, 手指, 腕表, 心电, ecg, ppg, photoplethysmography, light , electrode, finger

**C.    DOCUMENTS CONSIDERED TO BE RELEVANT**

| Category* | Citation of document, with indication, where appropriate, of the relevant passages | Relevant to claim No. |
| --- | --- | --- |
| X | CN 216495266 U (SHENZHEN GOODIX TECHNOLOGY CO., LTD.) 13 May 2022 (2022-05-13)<br>description, paragraphs 61-130, and figures 1-9 | 1-44 |
| A | CN 113171099 A (GOERTEK INC.) 27 July 2021 (2021-07-27)<br>entire document | 1-44 |
| A | CN 114257260 A (OPPO (CHONGQING) INTELLIGENT TECHNOLOGY CO., LTD.) 29 March 2022 (2022-03-29)<br>entire document | 1-44 |
| A | CN 212118134 U (SHENZHEN GOODIX TECHNOLOGY CO., LTD.) 11 December 2020 (2020-12-11)<br>entire document | 1-44 |
| A | CN 213217072 U (GUANGDONG OPPO MOBILE TELECOMMUNICATIONS CORP., LTD.) 18 May 2021 (2021-05-18)<br>entire document | 1-44 |
| A | KR 20190139440 A (H3 SYSTEM CO., LTD.) 18 December 2019 (2019-12-18)<br>entire document | 1-44 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **31 May 2024** | **05 June 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/078320** |

**C.     DOCUMENTS CONSIDERED TO BE RELEVANT**

| Category* | Citation of document, with indication, where appropriate, of the relevant passages | Relevant to claim No. |
|---|---|---|
| A | US 2023028745 A1 (MASIMO CORP.) 26 January 2023 (2023-01-26)<br>entire document | 1-44 |

Form PCT/ISA/210 (second sheet) (July 2022)

**INTERNATIONAL SEARCH REPORT**
Information on patent family members

International application No.

**PCT/CN2024/078320**

| Patent document cited in search report | | | Publication date (day/month/year) | Patent family member(s) | | | Publication date (day/month/year) |
|---|---|---|---|---|---|---|---|
| CN | 216495266 | U | 13 May 2022 | None | | | |
| CN | 113171099 | A | 27 July 2021 | None | | | |
| CN | 114257260 | A | 29 March 2022 | None | | | |
| CN | 212118134 | U | 11 December 2020 | None | | | |
| CN | 213217072 | U | 18 May 2021 | None | | | |
| KR | 20190139440 | A | 18 December 2019 | None | | | |
| US | 2023028745 | A1 | 26 January 2023 | KR | 20240032835 | A | 12 March 2024 |
| | | | | TW | 202310798 | A | 16 March 2023 |
| | | | | EP | 4370022 | A1 | 22 May 2024 |
| | | | | WO | 2023287789 | A1 | 19 January 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023104560482